(19) Europäisches Patentamt
European Patent Office
Office européen des brevets

(11) **EP 2 906 112 B1**

(12) **EUROPEAN PATENT SPECIFICATION**

(45) Date of publication and mention of the grant of the patent:
**15.03.2023 Bulletin 2023/11**

(21) Application number: **13846164.5**

(22) Date of filing: **14.10.2013**

(51) International Patent Classification (IPC):
***A61B 5/369*** *(2021.01)* ***A61B 5/00*** *(2006.01)*

(52) Cooperative Patent Classification (CPC):
**A61B 5/4821; A61B 5/369; A61B 5/4839; A61B 5/7235**

(86) International application number:
**PCT/US2013/064852**

(87) International publication number:
**WO 2014/059418 (17.04.2014 Gazette 2014/16)**

(54) **SYSTEM AND METHOD FOR MONITORING AND CONTROLLING A STATE OF A PATIENT DURING AND AFTER ADMINISTRATION OF ANESTHETIC COMPOUND**

SYSTEM UND VERFAHREN ZUR ÜBERWACHUNG UND STEUERUNG EINES ZUSTANDES EINES PATIENTEN WÄHREND UND NACH VERABREICHUNG EINER NARKOSEVERBINDUNG

SYSTÈME ET PROCÉDÉ DE SURVEILLANCE ET DE CONTRÔLE DE L'ÉTAT D'UN PATIENT PENDANT ET APRÈS L'ADMINISTRATION DE COMPOSÉ ANESTHÉSIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.10.2012 US 201261713267 P**

(43) Date of publication of application:
**19.08.2015 Bulletin 2015/34**

(73) Proprietor: **The General Hospital Corporation Boston, MA 02114 (US)**

(72) Inventors:
• **BROWN, Emery, N.**
  **Brookline, MA 02446 (US)**
• **PURDON, Patrick, L.**
  **Somerville, MA 02144 (US)**

(74) Representative: **Samson & Partner Patentanwälte mbB**
**Widenmayerstraße 6**
**80538 München (DE)**

(56) References cited:
EP-A2- 1 704 819    WO-A2-2004/037114
US-A- 6 067 467    US-A1- 2004 079 372
US-A1- 2004 193 068

**Description**

BACKGROUND OF THE INVENTION

The present disclosure generally relates to systems and method for monitoring and controlling a state of a patient and, more particularly, to systems and methods for monitoring and controlling a state of a patient receiving a dose of anesthetic compound(s) or, more colloquially, receiving a dose of "anesthesia."

Since 1846 and the first public uses of ether as a means to control pain during surgical procedures, anesthesia, analgesics, and other administered compounds to control pain and render patients unconscious have been a mainstay of medicine. However, while the use of the anesthetic and the number of compounds with anesthetic properties in clinical use have grown astronomically since the initial uses of ether, the scientific understanding of the operation of the body when under anesthesia is still developing. For example, a complete understanding of the effects of anesthesia on patients and operation of the patient's brain over the continuum of "levels" of anesthesia is still lacking. As such, anesthesiologists are trained to recognize the effects of anesthesia and extrapolate an estimate of the "level" of anesthetic influence on a given patient based on the identified effects of the administered anesthesia.

One common tool used by clinicians when monitoring patients receiving a dose of anesthesia is an electroencephalogram (EEG) system. EEG systems monitor electrophysiological signals of the brain. To provide the clinician with feedback, some EEG systems display a partial or amalgamized representation of the acquired signals as a waveform. However, as will be explained, many contemporary monitoring systems used during the administration of anesthesia provide feedback as a single dimensionless index that attempts to "quantify" the extremely-complex physiological responses of the patient receiving the dose of anesthesia and, thereby, convey the patient's depth of anesthesia. These EEG-based depth of anesthesia indices have been shown to poorly represent a patient's brain state, and moreover show substantial variability in underlying brain state and level of awareness at similar numerical values within and between patients. Recent advances in the neuroscience and neurophysiology of the anesthetic drugs show that different drugs act through different neural mechanism, producing different EEG signatures associated with different altered states of consciousness. These EEG signatures can vary across different anesthetic drugs. Analysis of the neural systems mechanisms for different anesthetic drugs can be found in Brown EN, Lydic R, & Schiff ND (2010) General anesthesia, sleep, and coma. New England Journal of Medicine 363(27):2638-2650 and in Brown EN, Purdon PL, & Van Dort CJ (2011) General anesthesia and altered states of arousal: a systems neuroscience analysis. Annual Review of Neuroscience 34:601-628. The different EEG signatures associated with different altered states of consciousness induced by the commonly-used anesthetic drug propofol can be found in Purdon PL, Pierce ET, Mukamel EA, Prerau MJ, Walsh JL, Wong KFK, Salazar-Gomez AF, Harrell PG, Sampson A, Cimenser A, Ching S, Kopell N, Tavares-Stoeckel CL, Habeeb K, Merhar R, Brown EN. Electroencephalogram signatures of loss and recovery of consciousness from propofol. Proceedings of the National Academy of Sciences, 2013 Mar 19;110(12):E1142-51. From the viewpoint of these recent advances, distinct anesthesia-related EEG signatures provide a more principled characterization of a patient's state under general anesthesia or sedation, and a more principled approach to controlling delivery of an anesthetic compound.

In practice, one common process that clinicians use is to monitor EEG display to identify indications of "burst suppression." Burst suppression is an example of an EEG pattern that can be observed when the brain has severely reduced levels of neuronal activity, metabolic rate, and oxygen consumption. For example, burst suppression is commonly seen in profound states of general anesthesia. One example of a profound state of a patient under general anesthesia is medical coma. The burst suppression pattern often manifests as periods of bursts of electrical activity alternating with periods during which the EEG is isoelectric or suppressed. A variety of clinical scenarios require medical coma for purposes of brain protection, including treatment of uncontrolled seizures-status epilepticus- and brain protection following traumatic or hypoxic brain injury, anoxic brain injuries, hypothermia, and certain developmental disorders. Burst suppression represents a specific brain state resulting from such injuries, disorders, or medical interventions.

Traditional systems and methods that attempt to quantify burst suppression proceeds in two steps. First, characteristics of burst suppression are identified in the acquired data and the burst and suppression events are segregated or separated from EEG artifacts. Second, these systems and methods attempt to quantify the "level" of burst suppression.

For example, commercially available brain monitoring devices like those produced by GE, Covidien, and Masimo, use a so-called "suppression ratio" as part of an algorithm to identify and track the state of burst suppression. These algorithms are focused on segmenting the EEG into bursts and suppression periods and then quantify the identified information.

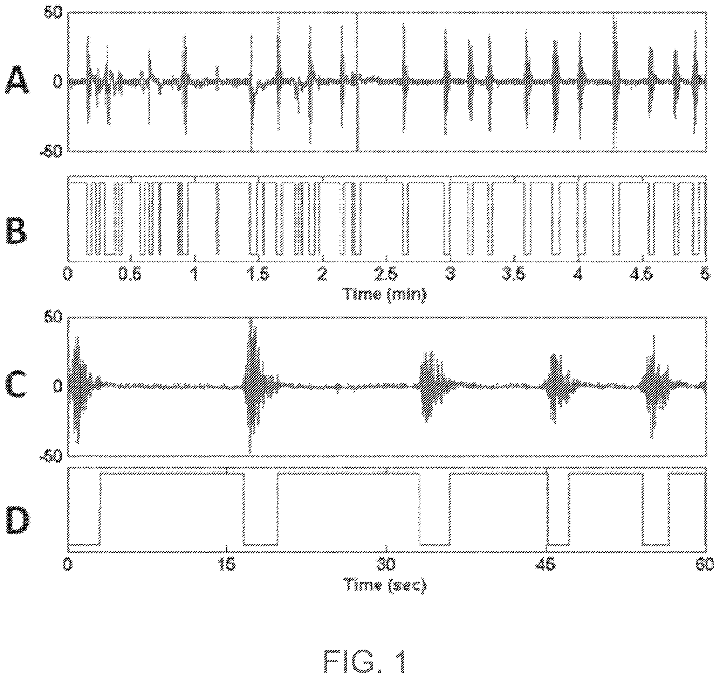

That is, several detection algorithms have been developed to accomplish the segregation or separation step. For example, many systems convert the EEG signal into a binary time series in which 1s correspond to suppression and 0s correspond to bursts. Figs.1 A and C, are EEG waveforms of respective 5-minute and a 1-minute segment illustrating burst suppression induced by the administration of the anesthetic propofol. Figs. 1 B and D show the binary series associated with the raw signals of Figs. 1A and C. Using this binary time series such as illustrated in Figs. 1B and D, these commercially-available systems attempt to "quantify" the level of burst suppression. One common method of quantification is called a "burst suppression ratio" (BSR). The BSR quantifies the proportion of time, in a given time interval, that the EEG signal is designated as being suppressed by the segmentation step. The BSR is a fraction, ranging from 0, meaning no suppression to 1, meaning isoelectric or flat EEG.

Systems implementing BSR as a means for quantifying a state of a patient have been studied and positively correlated with a reduction in cerebral metabolic rate (CMR). During general anesthesia and during induced hypothermia, a state of complete electrical silence will be reflected by a BSR of one and the CMR decreases in a dose-dependent manner until it plateaus at a constant rate.

Although the importance of quantitatively analyzing burst suppression using, for example, a metric like BSR is broadly appreciated, there are key shortcomings with current approaches. For example, even though the 0s and 1s can be computed on intervals as short as 100 msec or even every milli-second, it is not unusual to use several seconds of these binary values to compute the BSR. This assumes that the brain state remains stable throughout the period during which the BSR is being computed. When the level of brain activity is changing rapidly, such as with induction of general anesthesia, hypothermia, or with rapidly evolving disease states, this assumption does not hold true. Instead, the computation of the level of burst suppression should match the resolution at which the binary events are recorded. Unfortunately, this reflects a practical quandary for the algorithm designer. Namely, the design cannot calculate a BSR without a determined time interval, but the true interval would be best selected with knowledge of the BSR to be calculated.

To further compound the difficulties of using such BSR algorithms clinically, different manufactures use different segmentation algorithms to convert the EEG into a binary time-series. Accordingly, different devices from different manufactures produce different BSR estimates. Comparing results across devices/manufacturer's is often challenging. As a further clinical challenge, for any of the situations in which burst suppression is tracked quantitatively, an important objective is to make formal statistical comparisons at different points in time. However, the statistical properties of the BSR estimated by averaging the binary events over several second intervals have not been described. As a consequence, there is no principled way to use the current BSR estimates in formal statistical analyses of burst suppression. That is, there is a lack of formal statistical analyses and prescribed protocols to implement formal statistical analyses to be able to state with a prescribed level of certainty that two or more brain states differ using current BSR protocols.

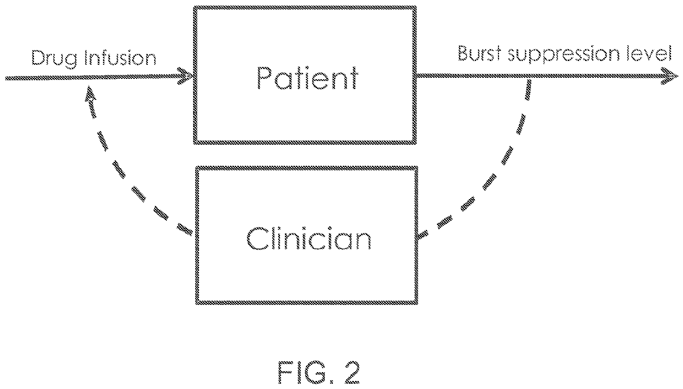

The shortcomings of these monitoring systems is compounded by the fact that they are often used as the information source on which clinicians make decisions. For example, referring to Fig. 2, a simplified schematic is illustrated showing that a "drug infusion" including a dose of anesthesia is delivered to a patient. Feedback from the patient is gathered by a monitoring system such as described above that attempts to identify and quantify burst suppression by providing an indication of "burst suppression level". The "burst suppression level" is generally the amount of burst suppression perceived by the clinician looking at the monitor display. This "burst suppression level" then serves as the input to a clinician that serves as the control of a feedback loop by adjusting the drug infusion levels based on the indicated "burst suppression level." This simplified example illustrates that errors or general inaccuracies in the "burst suppression level" indicated by the monitoring system and/or erroneous interpretations or assumptions by the clinician can exacerbate an already inexact process of controlling the drug infusion process. Such imprecision may be tolerable in some situations, but is highly unfavorable in others.

For example, in some clinical settings, it may be desirable to place a patient in a so-called "medical coma." To do so, burst suppression is induced by manually tuning drug infusion to meet certain specifications. Control of these infusions requires the nursing staff to monitor, frequency by eye, the infusion pump and the EEG waveform, and to titrate the infusion rate of the anesthetic drug to achieve and maintain the desired EEG pattern. It is impractical for the nursing staff to provide a continuous assessment of the EEG waveform in relation to the rate of drug infusion in such a way to maintain tight control of the patient's desired brain state.

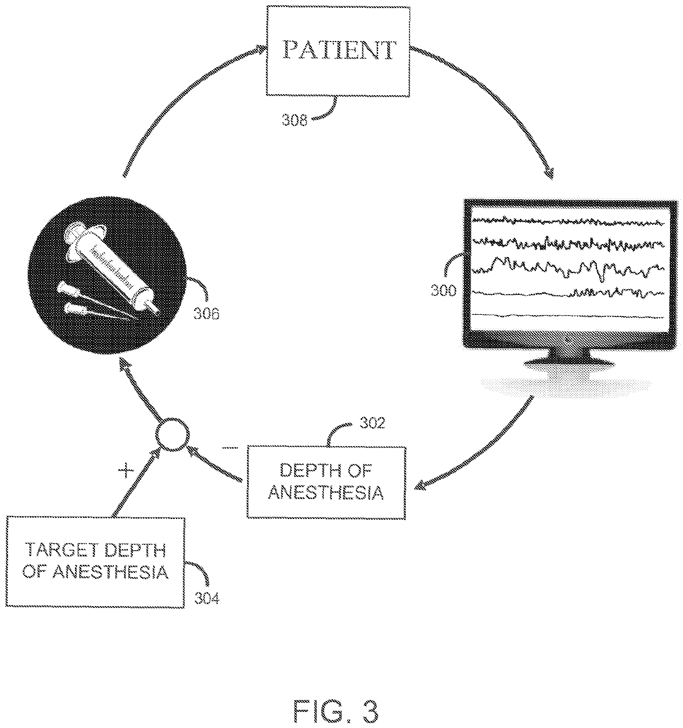

With these clinical challenges recognized, some have attempted to develop feedback and control systems the aid the clinician. For example, Bickford proposed an EEG-based, closed loop anesthetic delivery (CLAD) system more than 60 years ago. For example, a simplified schematic diagram of an early CLAD system is provided in Fig. 3. Bickford's original CLAD system of the 1950s used EEG content 300 in specific frequency bands as the control signal that indicated a current "depth of anesthesia" 302. The depth of anesthesia 302 was compared to a "target depth of anesthesia" 304, which determined with the drug infusion 306 should be increased or decreased. As such, a closed loop system was proposed to control the anesthetic delivered to the patient 308.

Later incarnations of the proposed CLAD systems used more sophisticated EEG analysis. For example, instead of simply relying on specific frequency bands as the control signal, systems were proposed that used metrics, such as the median frequency and the spectral edge, or the 50th and 95th quantiles of the power spectrogram, respectively. Studies observed a strong relationship between frequency content and its associated range and the corresponding depth of general anesthesia. Other possible control signals that were proposed included evoked potentials, or physiological responses, such as heart rate and blood pressure. Though commercial development of such systems did not begin in earnest until the 1980's, there have now been many clinical studies on the use of CLAD systems in anesthesiology practice and a system for sedation not using EEG is now commercially available.

Although CLAD systems have been around for many years and they are now used in anesthesiology practice outside of the United States, recent reports suggest that several problems with these systems have not been fully addressed. First, it has been recognized since 1937 that EEG patterns can serve as an indicator of brain state under general anesthesia. To date, sufficiently detailed quantitative analyses of the EEG waveform have not been performed to produce well-defined markers of how different anesthetic drugs or combinations of drugs alter the states of the patient and how such variations manifest in EEG waveforms and other physiological characteristics.

In an attempt to combat such problems, the so-called Bispectral index (BIS) has been used an EEG-based marker to track brain state under general anesthesia and to provide a control signal for CLAD systems. BIS is derived by computing spectral and bispectral features of the EEG waveform. The features are input to a proprietary algorithm to derive an index between 0 and 100, in which 100 correspond to fully awake state with no drug effects and 0 corresponds to the most profound state of coma. As referenced above, BIS often serves as a common, single indicator clinicians rely upon to interpret the data acquired by a monitoring system. That is, clinicians simply rely upon the BIS indication to make clinical decisions.

As a control signal, BIS can inherently have only limited success, as the same BIS value can be produced by multiple distinct brain states. A patient under general anesthesia with isoflurane and oxygen, a patient sedated with dexmedetomidine, and a patient in stage III, or slow-wave, sleep can all have BIS values in the 40-to-60 range, which is the BIS interval in which surgery is conducted. Of these three patients, only the first is most likely in a state of "general anesthesia" and appropriate for conducting surgery. In this context, "general anesthesia" refers to unconsciousness, amnesia, analgesia, akinesia with maintenance of physiological stability. Similarly, patients anesthetized with ketamine alone or in combination with other anesthetic agents show high BIS values suggesting an awake or lightly sedated state, despite being in a state of general anesthesia. Although most reports nonetheless claim successful brain state control, such control has not been reliably demonstrated in individual subjects in a study or patients in real-time.

Second, using BIS to account for individual variability in response to anesthetic drugs and hence, in EEG patterns, under normal, surgical, and intensive care unit conditions is a challenge. Third, EEG processing by commercially-available monitors of anesthetic state is performed, not in real-time, but with a 20-to-30-second delay. Fourth, CLAD systems use ad-hoc algorithms instead of formal deterministic or stochastic control paradigms in their design. As a consequence, the reports in which CLAD systems have been implemented do not show reliable repeatable control results. Indeed, to give the appearance of successful control, the results of several subjects are often averaged in plots of CLAD performance. Finally, some have proposed the theoretical use of established control principles to design a CLAD system. However, such proposals have suggested the derivation of a wavelet-based index of anesthetic depth from the EEG, which fundamentally proposes a control signal that is analogous to BIS. Simply, until more is known about the neurophysiology of how EEG patterns relate to brain states under general anesthesia, developing generally applicable CLAD systems is a challenging problem. To this point, as described above, metrics such as BSR suffer from similar limitations and, thus, have not been suitable for developing generally applicable CLAD systems for at least the reasons discussed above.

Perhaps recognizing the complex nature of the EEG waveform and the shortcomings of BIS as a control system, Vijn and Sneyd designed CLAD systems for rats using a different metric, namely burst suppression ratio (BSR), as the control signal. BSR, is defined as the proportion of time per epoch that the EEG is suppressed below a predetermined voltage threshold. The BSR ranges from 0, meaning no suppression, to 1, meaning an isoelectric EEG. The objective of such investigation was to develop a model-free approach to CLAD-system design to determine if performance of new drugs in a CLAD system could provide useful information on drug design. They processed their error signal using a non-standard deterministic control strategy that was the product of a proportional and an integral term. Although the authors claim that their CLAD system maintained control of BSR for both propofol and etomidate, they reported BSR time courses averaged over groups of rats and not for individual animals. The Vijn and Sneyd CLAD system was recently implemented by Cotten et al. to test the efficacy of new etomidate-based anesthetics in controlling BSR in rats. These authors also reported only average time courses. Accordingly there seems to be a lack of studies on the use of CLAD systems to control burst suppression in human experiments or in the ICU to maintain a level of medical coma.

To further complicate matters, there are a great number of variables that can influence the effects, effectiveness, and, associated therewith, the "level" of anesthetic influence on a given patient. Thus, closed-loop control systems can fail if the drug infusion does not account for any of the plethora of variables. Some variables include physical attributes of the patient, such as age, state of general health, height, or weight, but also less obvious variables that are extrapolated, for example, based on prior experiences of the patient when under anesthesia. When these variables are compounded with the variables of a given control system or method and the variables presented by a particular anesthetic compound or, more so, combination of anesthetic compounds, the proper and effective administration of anesthesia to a given patient can appear to be an art, rather than a science.

In addition, whether controlled by a system, such as a CLAD system, or a more traditional clinician-specific control, emergence from general anesthesia is a slow passive process achieved simply by allowing the effects of the drug to wear off. Emergence from anesthesia is traditionally a passive process whereby anesthetic drugs are merely discontinued at the end of surgery, and no drugs are administered to actively reverse their effects on the brain and central nervous system. That is, the general anesthetic agents are merely discontinued at the end of surgery, leaving the anesthesiologist and surgeon to wait for the patient to recover consciousness. The timing of emergence can be unpredictable because many factors including the nature and duration of the surgery, and the age, physical condition and body habitus of the patient, can greatly affect the pharmacokinetics and pharmacodynamics of general anesthetics. Although the actions of many drugs used in anesthesiology can be pharmacologically reversed when no longer desired (e.g. muscle relaxants, opioids, benzodiazepines, and anticoagulants), this is not the case for general anesthetic induced loss of consciousness. While some basic ideas for actively reversing the effects of anesthesia have been considered, they do not translate well to traditional monitoring systems and control methods because these monitoring and control methods are generally unidirectional. For example, using burst-suppression based metrics for determining an increasing state of consciousness is counterintuitive, at best. Not surprisingly, then, control algorithms have not been developed to facilitate actively controlled recovery.

Considering the above, there continues to be a clear need for systems and methods to accurately monitor and quantify patient states and based thereon, provide systems and methods for controlling patient states during administration of anesthetic compounds.

Document US 2004/079372 A1 discloses a method for monitoring anesthetization of a patient by connecting a plurality of electrodes to the scalp of the patient and administering sufficient anesthesia selected by an operator. The brain waves are amplified and digitized after the patient has been anesthetized before and during the medical procedure to obtain two sets of digital data. The data sets are analyzed in time and frequency domains and separate trajectories are computed for at least two different indices of an anesthetic state of the patient during the medical procedure.

Document US 2004/193068 A1 discloses a system with improved accuracy in monitoring, analyzing, detecting, predicting and/or providing alerts and alarms associated with depth of anaesthesia, depth of consciousness, hypnotic state, sedation depth, fatigue or vigilance of a subject, with as few as 3 surface electrodes. The systems incorporate real-time phase, amplitude and frequency analysis of a subject's encephalogram and weight outputs of various types of analyses to produce an integrated analysis or display for precise indication or alert to users of the systems including anaesthetists, nurses and other medical personnel, transport drivers and machine workers.

SUMMARY OF THE INVENTION

The present invention is defined in the appended independent claims, and overcomes drawbacks of previous technologies by providing systems and methods that provide a number of advantages and capabilities not contemplated by, recognized in, or possible with traditional systems or known-methodologies related to the administration and control of anesthetic compounds. Further detailed embodiments are described in the dependent claims. The embodiments or examples of the following description which are not covered by the appended claims are considered as not being part of the invention according to this description.

In one embodiment, a system and method for monitoring and controlling the administration of at least one drug having anesthetic properties are provided. The system includes a plurality of sensors configured to acquire physiological data from the patient, and a user interface configured to receive an indication of at least one of a characteristic of the patient and the at least one drug having anesthetic properties. The system also includes at least one processor configured to review the physiological data from the plurality of sensors and the indication from the user interface. The processor is also configured to assemble the physiological data into sets of time-series data, analyze the sets of time-series data to determine signature profiles consistent with the administration of at least one drug having anesthetic properties and identify, using signature profiles, at least one of a current state and a predicted future state of the patient based on the indication. The processor is further configured to generate a report indicating at least one of the current state and the predicted future state of the patient induced by the drug.

In another embodiment, a method is provided for monitoring a patient experiencing an administration of at least one drug having anesthetic properties. The method includes arranging a plurality of sensors configured to acquire physiological data from a patient and reviewing the physiological data from the plurality of sensors and the indication from the user interface. The method also includes assembling the physiological data into sets of time-series data and analyzing the sets of time-series data to determine signature profiles consistent with the administration of at least one drug having anesthetic properties. The method further includes identifying using signature profiles at least one of a current state and a predicted future state of the patient, based on the indication and generating a report including information regarding at least one of the current state and the predicted future state of the patient induced by the drug.

In yet another embodiment, a system is provided for monitoring and controlling a patient experiencing an administration of at least one drug having anesthetic properties. The system includes a plurality of sensors configured to acquire physiological data from the patient and a user interface configured to receive an indication of at least one of a characteristic of the patient and the at least one drug having anesthetic properties. The system also includes at least one processor configured to review the physiological data from the plurality of sensors and the indication from the user interface. The processor is also configured to assemble the physiological data into sets of time-series data and analyze the sets of time-series data to determine signature profiles consistent with the administration of at least one drug having anesthetic properties. The processor is further configured to identify, using signature profiles, at least one of a current state and a predicted future state of the patient based on the indication, and control the administration of the least one drug to attain the predicted future state. The processor is further configured to generate a report indicating at least one of the current state and the predicted future state of the patient induced by the drug.

The foregoing and other advantages of the invention will appear from the following description. In the description, reference is made to the accompanying drawings which form a part hereof, and in which there is shown by way of illustration a preferred embodiment of the invention. Such embodiment does not necessarily represent the full scope of the invention, however, and reference is made therefore to the claims and herein for interpreting the scope of the invention.

BRIEF DESCRIPTION OF THE DRAWINGS

The present invention will hereafter be described with reference to the accompanying drawings, wherein like reference numerals denote like elements.

Fig. 1A is an EEG waveform illustrating a burst suppression pattern over a 5 minute interval taken from a patient following a propofol bolus induction.

Fig. 1B is an EEG waveform illustrating a corresponding binary signal to the pattern of Fig. 1A where 1 represents a suppression and 0 represents a burst.

Fig. 1C is an EEG waveform illustrating a one minute segment taken from the pattern in Fig. 1A.

Fig. 1D is an EEG waveform illustrating a corresponding binary signal to the pattern of Fig. 1C.

Fig. 2 is a schematic block diagram of a traditional anesthetic compound monitoring and control system that depends completely upon a clinician.

Fig. 3 is a schematic illustration of a traditional closed-loop anesthesia delivery (CLAD) system.

Fig. 4 is an illustration of a close-loop monitoring and control system in accordance with the present invention.

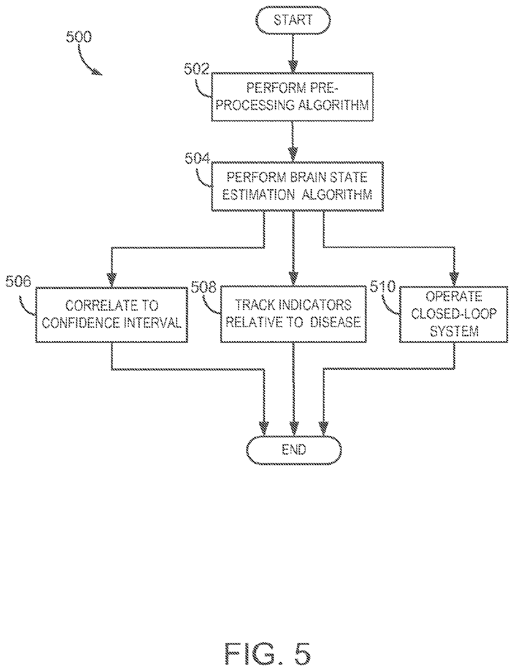

Fig. 5 is a flow chart setting forth the steps of a monitoring and control process in accordance with the present invention, for example, as may be implemented using a system such as described with respect to Fig. 4.

Fig. 6 is a flow chart setting forth steps of a control method in accordance with the present invention.

Fig. 7 is an illustration of a further close-loop monitoring and control system in accordance with the present invention.

Fig. 8 is a flow chart setting forth the steps of an expectation maximization (EM) algorithm in accordance with the present invention.

Fig. 9A is a schematic illustration of a two-compartment pharmacokinetics model in accordance with the present invention.

Fig. 9B illustrates a waveform subjected to a thresholding algorithm in accordance with the present invention to yield a binary waveform for a binary or BSP filter.

Fig. 10 is a block diagram of a closed-loop monitoring and control system in accordance with the present invention.

Fig.11A is an EEG waveform of a Sprague-Dawley rat during an experiment testing the effect of physostigmine on burst suppression.

Fig. 11B. is a plot illustrating a binary signal associated with the EEG of Fig. 11A.

Fig. 11C is a BSR estimate waveform associated with the EEG waveform of Fig. 11A and computed in one second epochs.

Fig.11D is a BSP estimate waveform associated with the EEG of Fig. 11A and computed with a binary smoother in one second epochs.

Fig. 12A is a BSP estimate waveform of a Sprague-Dawley rat with confidence intervals computed in one second epochs.

Fig. 12B is a point-by-point BSP comparison matrix.

Fig. 13A is an EEG waveform recorded in a patient undergoing hypothermic arrest.

Fig 13B is a binary signal associated with the EEG of Fig. 13A.

Fig. 13C is a BSR estimate waveform associated with the EEG of Fig. 13A and computed in one second epochs.

Fig. 13D is a BSP estimate waveform associated with the EEG of Fig. 13A and computed with the binary filter in one second epochs.

Fig. 13E is a BSP estimate waveform associated with the EEG of Fig. 13A after use of the smoothing algorithm.

Fig. 14A is a recorded EEG waveform of a patient following a propofol bolus induction for surgery.

Fig. 14B is a binary signal associated with the EEG of Fig. 14A.

Fig. 14C is a BSR estimate waveform associated with the EEG of Fig. 14A and computed in one second epochs.

Fig. 14D is a BSP estimate waveform associated with the EEG of Fig. 14A and computed with the binary filter in one second epochs.

Fig. 14E is a BSP estimate waveform associated with the EEG of Fig. 14A after use of the smoothing algorithm.

Fig. 15A is a simulated BSP time course after a 10-second bolus of 8 mg kg$^{-1}$ propofol in the optimized rat two-compartment model.

Fig. 15B is a simulated BSP time course after a 10-second bolus of 3.5 mg kg$^{-1}$ etomidate in the optimized rat two-compartment model.

Fig. 16A shows the time courses for a bolus dose of propofol in rats illustrating the accuracy of binary or BSP filter as tested on simulated dynamic data.

Fig. 16B shows the time courses for bolus dose of etomidate in rats illustrating the accuracy of binary or BSP filter as tested on simulated dynamic data.

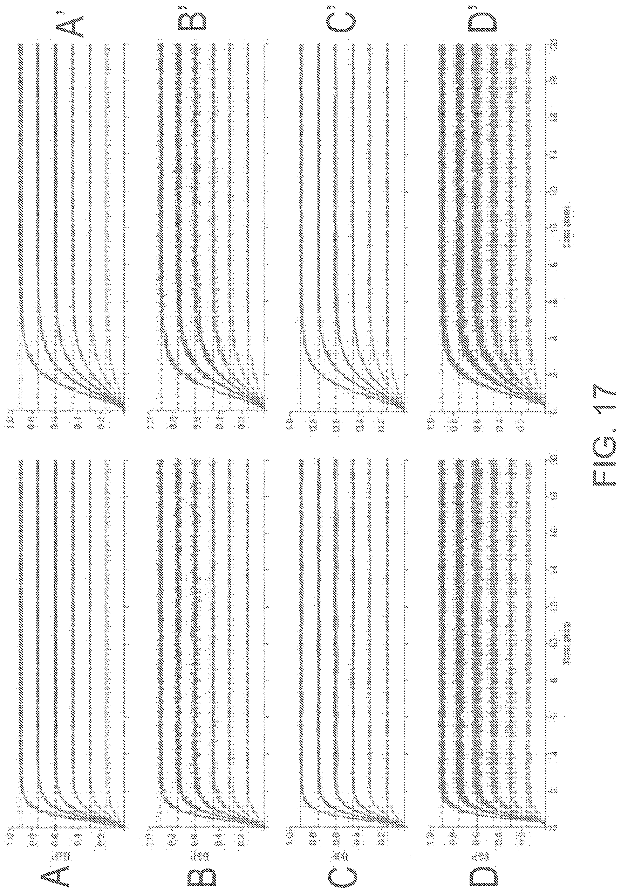

Figs. 17A-17D' are simulations at six targets for infusion of propofol 17A-17D and etomidate 17A'-17D' in rats. Figs. 17A and 17A' are an ideal deterministic scenario in which the feedback is the numerically evaluated $x_{2t}$ transformed to $p_t$. Figs. 17B and 17B' are a noisy deterministic scenario in which the feedback is the numerically evaluated $x_{2t}$ transformed to $p_t$ with Gaussian relative error of standard deviation 3% added. Figs. 17C and 17C' are BSP time courses as evaluated from the underlying pharmacokinetics models. Ten simulations at each target were run in the stochastic scenario, in which the feedback is the binary or BSP filter's output, $\hat{p}_{t|t}$. Figs. 17D and 17D' are ten time courses at each target of the observable $\hat{p}_{t|t}$ feedback signal.

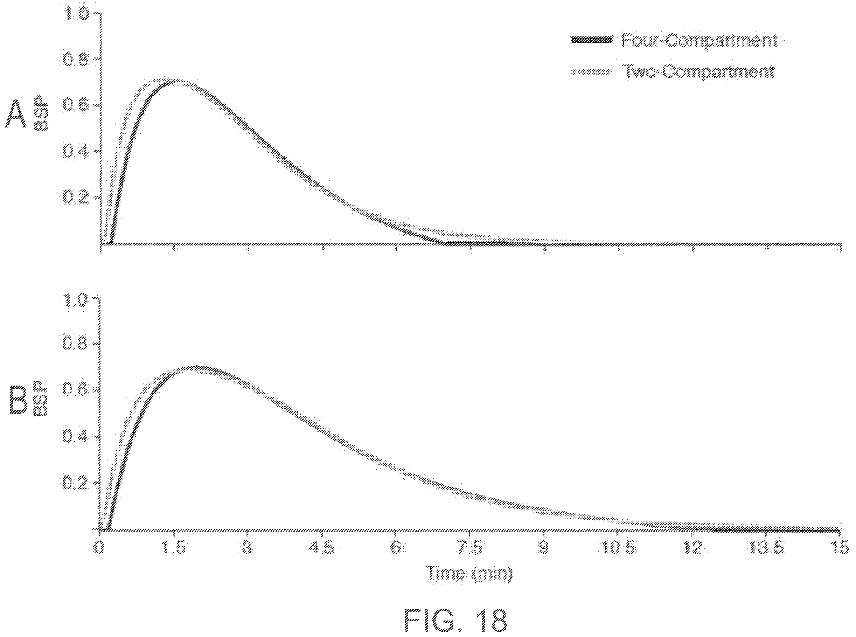

Fig. 18A is a simulated BSP time course after a 10-second bolus of 250 mg kg$^{-1}$ propofol in optimized human two-compartment pharmacokinetic model compared to four-compartment model.

Fig. 18B is a simulated BSP time course after a 10-second bolus of 110 mg kg$^{-1}$ etomidate in optimized human two-compartment model compared to four-compartment model.

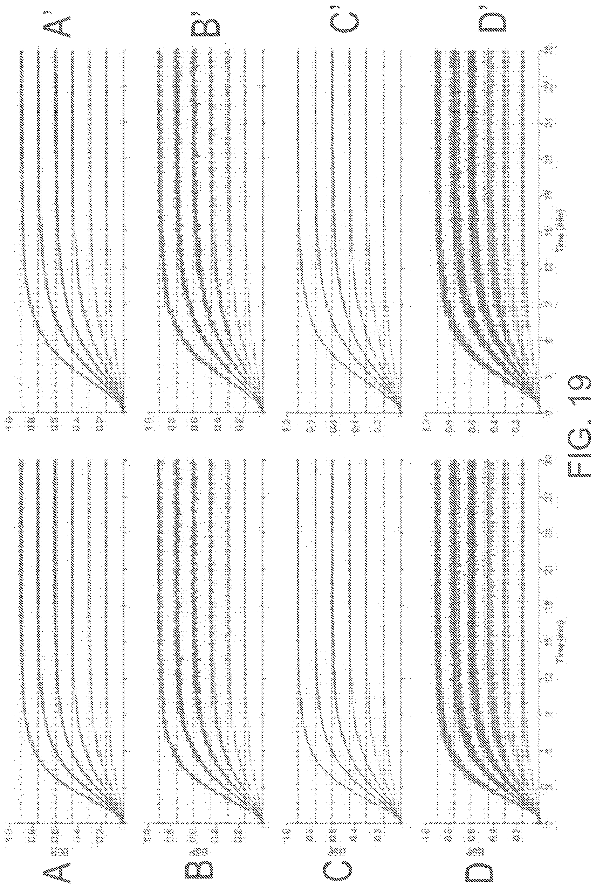

Figs. 19A-D' are simulations at six targets for infusion of propofol 19A-19D and etomidate 19A'-19D' in humans. Figs. 19A and 19A' are an ideal deterministic scenario in which the feedback is the numerically evaluated $x_{2t}$ transformed to $p_t$. Figs. 19B and 19B' are a noisy deterministic scenario in which the feedback is the numerically evaluated $x_{2t}$ transformed to $p_t$, with Gaussian relative error of standard deviation 3% added. Figs. 19C and 19C' are BSP time courses as evaluated from the underlying pharmacokinetics models. Ten simulations at each target were run in the stochastic scenario, in which the feedback is the binary or BSP filter's output, $\hat{p}_{t|t}$. Fig. 19D and 19D' are ten time courses at each target of the observable $\hat{p}_{t|t}$ feedback signal.

Figs. 20A and 20B are time courses of propofol infusion in the human model simulated with a changing BSP target. Fig. 20A shows the simulated observable stochastic signal with a changing BSP target, namely 0.3, 0.8, 0.6, 0.1, and 0. Fig. 20B shows the infusion rate, as calculated by the proportional-integral controller, corresponding to the variable-target simulation.

## DETAILED DESCRIPTION

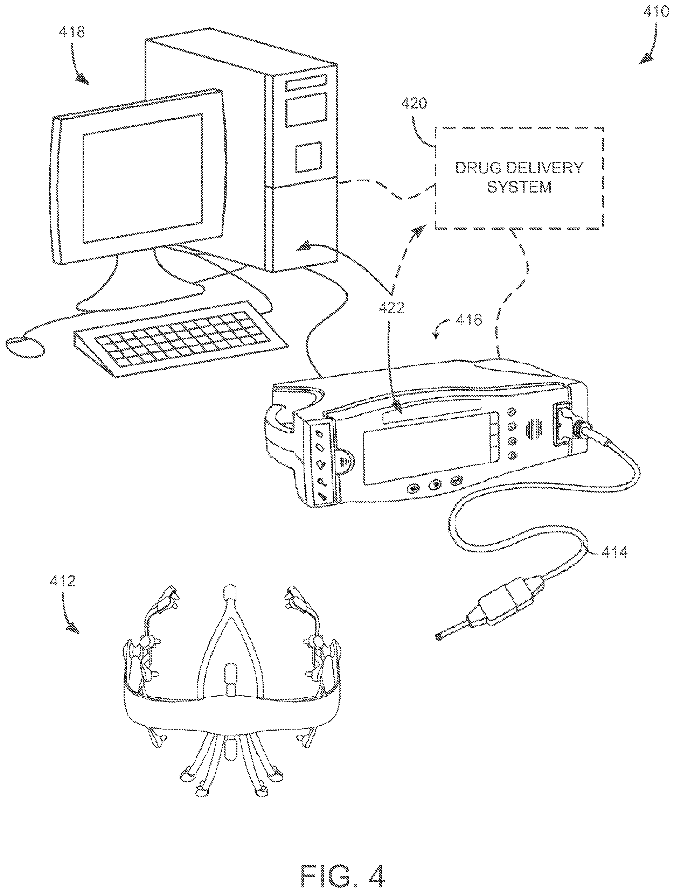

In one embodiment of the present invention, systems and methods for monitoring and controlling a state of a patient during and after administration of an anesthetic compound or compounds are provided. Specifically, referring to Fig. 4, an exemplary system 410 in accordance with the present invention is illustrated. The system 410 includes a patient monitoring device 412, such as a physiological monitoring device, illustrated in Fig. 4 as an electroencephalography (EEG) electrode array. However, it is contemplated that the patient monitoring device 412 may also include mechanisms for monitoring galvanic skin response (GSR), for example, to measure arousal to external stimuli or other monitoring system such as cardiovascular monitors, including electrocardiographic and blood pressure monitors, and also ocular microtremor monitors. One specific realization of this design utilizes a frontal Laplacian EEG electrode layout with additional electrodes to measure GSR waveforms from an EEG monitoring system. Specifically, at process block 502, identified indicators, such as burst and suppression intervals in the EEG waveform, are converted into a binary time series. This time series is preferably a "real-time" series that, at process block 504, is provided as an input into the BSP algorithm, which provides a second-to-second estimate of the brain's state of burst suppression using the concept of a state space model for binary and point process observations.

As will be described in greater detail, the brain state estimation algorithm output, at process block 506, is correlated with "confidence intervals." The confidence intervals are predicated on formal statistical comparisons between the brain state estimated at any two time points. Also, at process block 508, the output of the brain state estimation algorithm can be used to identify and track brain state indicators, such as burst suppression, during medical procedures or disease states. Exemplary medically-significant states include hypothermia, general anesthesia, medical coma, and sedation to name but a few. The output of the brain state estimation algorithm can further be used, at process block 510 as part of a closed-loop anesthesia control process.

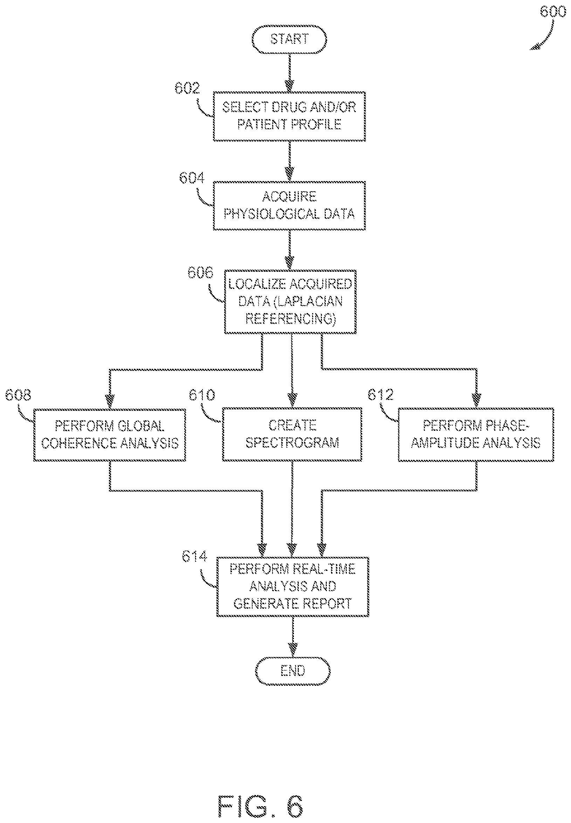

In another embodiment, the present invention provides a system and method for analysis and reporting. Referring to Fig. 6, the process 600 begins at process block 602 with the selection of a desired drug, such as anesthesia compound or compounds, and/or a particular patient profile, such as a patient's age height, weight, gender, or the like. Furthermore, drug administration information, such as timing, dose, rate, and the like, in conjunction with the above-described EEG data may be acquired and used to estimate and predict future patient states in accordance with the present invention. As will be described, the present invention recognizes that the physiological responses to anesthesia vary based on the specific compound or compounds administered, as well as the patient profile. For example, elderly patients have a tendency to show lower amplitude alpha power under anesthesia, with some showing no visible alpha power in the unconscious state. The present invention accounts for this variation between an elderly patient and a younger patient. Furthermore, the present invention recognizes that analyzing physiological data for signatures particular to a specific anesthetic compound or compounds administered and/or the profile of the patient substantially increases the ability to identify particular indicators of the patient's brain being in a particular state and the accuracy of state indicators and predictions based on those indicators.

For example, the following drugs are examples of drugs or anesthetic compounds that may be used with the present invention: Propofol, Etomidate, Barbiturates, Thiopental, Pentobarbital, Phenobarbital, Methohexital, Benzodiazepines, Midazolam, Diazepam, Lorazepam, Dexmedetomidine, Ketamine, Sevoflurane, Isoflurane, Desflurane, Remifenanil, Fentanyl, Sufentanil, Alfentanil, and the like. However, the present invention recognizes that each of these drugs, induces very different characteristics or signatures, for example, within EEG data or waveforms.

With the proper drug or drugs and/or patient profile selected, acquisition of physiological data begins at process block 604, for example, using a system such as described with respect to Fig. 4, where the acquired data is EEG data. The present invention provides systems and methods for analyzing acquired physiological information from a patient, analyzing the information and the key indicators included therein, and extrapolating information regarding a current and/or predicted future state of the patient. To do so, rather than evaluate physiological data in the abstract, the physiological data is processed. Processing can be done in the electrode or sensor space or extrapolated to the locations in the brain. As will be described, the present invention enables the tracking of the spatiotemporal dynamics of the brain by combining additional analysis tools, including, for example, spectrogram, phase-amplitude modulation, coherence, and global coherence analyses. As will be apparent, reference to "spectrogram" may refer to a visual representation of frequency domain information.

Laplacian referencing can be performed at process block 606 to estimate radial current densities perpendicular to the scalp at each electrode site of, for example, the monitoring device of Fig. 4. This may be achieved by taking a difference between voltages recorded at an electrode site and an average of the voltage recorded at the electrode sites in a local neighborhood. Other combinations of information across the plurality of electrodes may also be used to enhance estimation of relevant brain states. Next, process blocks 608 and 610 yield two pieces of valuable information, namely, the spectrogram and global coherence information, which show different spatiotemporal activity at different states of the patient receiving anesthesia. Though "spectrogram" processing is performed, a visual representation of the spectrogram need not be displayed. In one example, for propofol, when patients are awake, the spectrograms will show strong occipital $\alpha$ activity. After loss of consciousness, the spectrograms will show a loss of $\alpha$ activity and an increase in $\delta$ activity, in the occipital sites and strong $\alpha$ and $\delta$ activity in the frontal sites. Increased power in the $\alpha$ (8-14 Hz), $\beta$ (12-30 Hz), and $\delta$ (1-4 Hz) ranges in the frontal sites will occur after loss of consciousness, consistent with the well-known pattern of anteriorization. As patients lose responsiveness, the coordinated activity over the occipital sites in the $\alpha$ range diminish. When patients are unconscious, strong coordinated activity in the $\alpha$ range is observed broadly over the frontal electrode sites at which the spectrograms show the anteriorization pattern. Despite the overall high $\delta$ activity in the spectrograms, coordinated activity may only be observed in the $\alpha$ range. The relative power in the occipital $\alpha$ and $\delta$ ranges reliably track the patients' behavioral responses. For propofol, the occipital $\alpha$ power is greater than the $\delta$ power when the patient is awake, and the reverse is true when the patients are unconscious. The strong global coherence in the $\alpha$ range indicates highly coordinated activity in the frontal electrode sites. Thus, global coherence and weight matrices along with spectrograms provide a first level of data for determining a current state and predicting a future state of a patient's brain under anesthesia. Spectrograms and related coherence and global coherence estimates could be made using the multitaper method to achieve precise and specific timefrequency resolution and efficiency properties necessary to estimate the relevant brain states. Further details regarding initial testing and validation of such processes are provided in Cimenser A, Purdon PL, Pierce ET, Walsh JL, Salazar-Gomez AF, Harrell PG, Tavares-Stoeckel C, Habeeb K, Brown EN (2011) Tracking brain states under general anesthesia by using global coherence analysis. Proceedings of the National Academy of Sciences of the United States of America 108:8832-8837. Other signals may likewise be tracked, such as global coherence and phase-amplitude modulation.

At process block 612, phase-amplitude analysis is performed that considers the amplitude of a given signal with respect to the phase of other signals and vice versa. As explained above, spectral analysis of EEG recordings allows the present invention to track systematic changes in the power in specific frequency bands associated with administration of anesthesia, including changes in $\delta$ (1-4 Hz), $\theta$ (5-8 Hz), $\alpha$ (8-14 Hz), $\beta$ (12-30 Hz), and $\gamma$ (30-80 Hz). However, spectral analysis treats oscillations within each frequency band independently, ignoring correlations in either phase or amplitude between rhythms at different frequencies.

The above-described selection of an appropriate analysis context based on a selected drug or drugs (process block 602), the acquisition of data (process block 604), and the analysis of the acquired data (process blocks 608-612) set the stage for the new and substantially improved real-time analysis and reporting on the state of a patient's brain as an anesthetic or combination of anesthetics is being administered and the recovery from the administered anesthetic or combination of anesthetics occurs, the administration of an anesthetic or combination of anesthetics not being part of the invention as claimed. That is, although, as explained above, particular indications or signatures related to the states of effectiveness of an administered anesthetic compound or anesthetic compounds can be determined from each of the above-described analyses (particularly, when adjusted for a particular selected drug or drugs), a mechanism for considering each of these separate pieces of data and more to accurately indicate and/or report on a state of the patient under anesthesia and/or the indicators or signatures that indicate the state of the patient under anesthesia is provided.

Specifically, referring to process block 614, any and all of the above-described analysis and/or results can be reported and, in addition, can be coupled with a precise statistical characterizations of behavioral dynamics. That is, behavioral dynamics, such as the points of loss-of-consciousness and recovery-of-consciousness can be precisely, and statistically calculated and indicated in accordance with the present invention. To do so, the present invention may use dynamic Bayesian methods that allow accurate alignment of the spectral and global coherence analyses relative to behavioral markers.

As stated above, the present invention is not only able to control the administration of anesthetic compounds for the purpose of placing the patient in a state of reduced consciousness influenced by the anesthetic compounds, such as "medical coma," but can implement and reflect systems and methods for bringing a patient to and from a state of greater or lesser consciousness.

Prior to the above-referenced work in the co-pending application, efforts utilized the classic approach of developing drugs that antagonize the actions of general anesthetics at the molecular level. However, such efforts have not been feasible because of the lack of clearly defined molecular targets through which general anesthetics induce loss of consciousness. The present disclosure recognizes, instead, that at the level of neural circuits and systems, there are arousal pathways that can be utilized to actively induce emergence from general anesthesia.

For example, in accordance with one aspect of the present invention, methylphenidate can be used as an inhibitor of dopamine and norepinephrine reuptake transporters and actively induces emergence from isoflurane general anesthesia. Methylphenidate can be used to restore consciousness, induce electroencephalogram changes consistent with arousal, and increase respiratory drive. The behavioral and respiratory effects induced by methylphenidate can be inhibited by droperidol, supporting the evidence that methylphenidate induces arousal by activating a dopaminergic arousal pathway. Plethysmography and blood gas experiments establish that methylphenidate increases minute ventilation, which increases the rate of anesthetic elimination from the brain. These and other findings are included in Solt et al., "Methylphenidate Actively Induces Emergence from General Anesthesia," Anesthesiology 2011; 115:791- 803.

With the above as background, the present invention establishes that methylphenidate or other agents can be used to actively induce emergence from isoflurane, propofol, or other general anesthesia by increasing arousal using a control system, such as described above. For example, in addition to the explanation above, Chemali et al., "Active Emergence from Propofol General Anesthesia Is Induced by Methylphenidate,"Anesthesiology 2012; 116:998 -1005, describes the use of methylphenidate perform active emergence from the use of propofol as a general anesthesia.

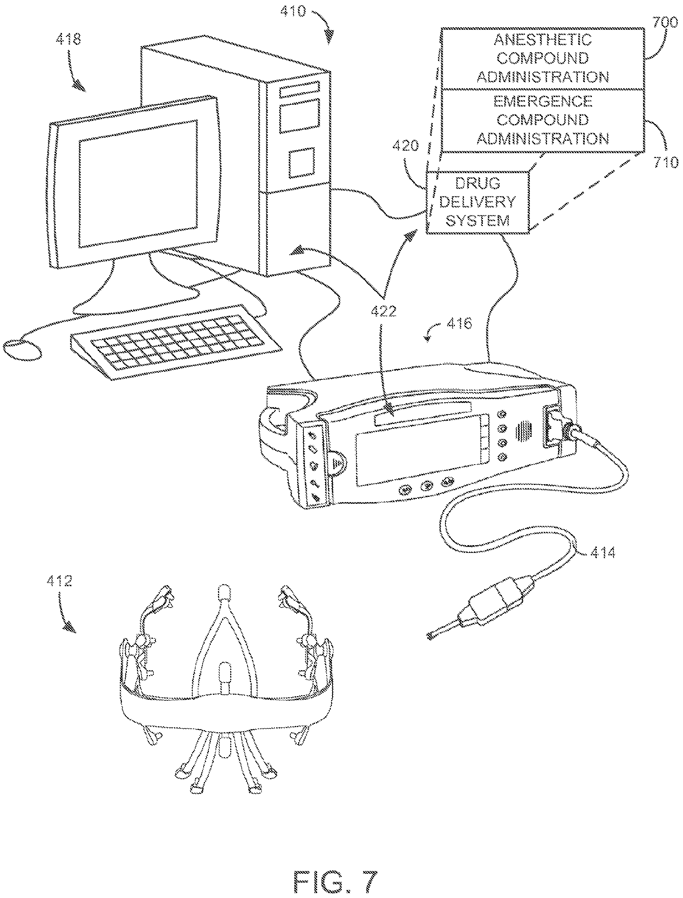

Specifically, a system such as described above with respect to Fig. 4 can be provided to carry out active emergence from anesthesia. That is, such a system can be integrated with the system 410 of Fig. 4. Referring to Fig. 7, the drug delivery system 420 may include two specific sub-systems. Specifically, the drug delivery system 420 may include an anesthetic compound administration system 700 that is designed to deliver doses of one or more anesthetic compounds to a subject, as described in detail above. The drug delivery system 420 may also include a emergence compound administration system 710 that is designed to deliver doses of one or more compounds that will reverse general anesthesia or the enhance the natural emergence of a subject from anesthesia. For example, methylphenidate (MPH) and analogues and derivatives thereof induces emergence of a subject from anesthesia-induced unconsciousness by increasing arousal and respiratory drive. Thus, the emergence compound administration system 710 can be used to deliver methylphenidate (MPH), amphetamine, modafinil, amantadine, or caffeine to reverse general anesthetic-induced unconsciousness and respiratory depression at the end of surgery. The MPH may be dextro-methylphenidate (D-MPH), racemic methylphenidate, or leva-methylphenidate (L-MPH), or may be compositions in equal or different ratios, such as about 50%:50%, or about 60%:40%, or about 70%:30%, or 80%:20%, 90%:10%,95%:5% and the like. Other agents may be administered as a higher dose of methylphenidate than the dose used for the treatment of Attention Deficit Disorder (ADD) or Attention Deficit Hyperactivity Disorder (ADHD), such as a dose of methylphenidate can be between about 10mg/kg and about 5mg/kg, and any integer between about 5mg/kg and 10mg/kg. In some situations, the dose is between about 7mg/kg and about 0.1mg/kg, or between about 5mg/kg and about 0.5mg/kg. Other agents may include those that are inhaled.

In yet another embodiment, a metric or a plurality of metrics are monitored by the system 410, to facilitate accurate monitoring and/or control. For example, as discussed above, one clinically-relevant phenomenon is "burst suppression." In accordance with one aspect of the present invention, a new state space model has been developed to conduct dynamic analysis of burst suppression. However, instead of traditional metrics for monitoring bust suppression, such as BSR, the present invention introduces the concept of the burst suppression probability (BSP). BSP can be used to interpret the brain's instantaneous likelihood of being in the suppressed state. The aforementioned system 410 may implement a BSP filter algorithm to track burst-suppression in real-time and a smoothing algorithm to analyze burst suppression recorded in a fixed time interval. As will be described, one such approach enables the tracking of burst suppression on a second-to-second time scale and the system 410 can make formal statistical comparisons of this activity at different times.

Specifically, the BSP algorithm is based on a state space framework for point processes and binary observations. The observation model is a binomial process and the temporal evolution of the brain state of burst suppression is defined by a state equation represented as a Gaussian random walk. By making a logistic transformation on the state, the concept of the BSP is introduced to define the brain's state of burst suppression. In accordance with one configuration, the model is estimated using an approximate expectation maximization (EM) algorithm and illustrates its application in the analysis of rodent burst suppression recordings under general anesthesia, a patient emerging from hypothermia, and a patient following induction of general anesthesia. The approach of the present invention obviates the need to artificially average "BSR" over long epochs and allows formal statistical comparisons of burst activity at different time points. The state-space model suggests a more principled and informative way to analyze this important EEG brain state, as will be described in more detail below.

*Brain State Estimation Model*

To formulate the state space model in accordance with the present invention, a state space paradigm for analyzing point processes, bust suppression information, and other general binary time series can be used. It can be assumed that EEG data collected over an observation interval $(0, T]$ and the state space model is defined on a discrete set of lattice points within that interval. To define the lattice, a number ($I$), preferably a large number, is chosen and the interval is divided into $I$ subintervals of equal width $\Delta = TI^{-1}$. The state space model is evaluated at $i\Delta$ for $i = 1...I$.

A state-space model may is characterized by its state and observation equations. The state equation defines the unobservable state process whose evolution we wish to track over time. In one aspect of the present invention, the state represents the brain's state of burst suppression. For example, the state can be defined to be positively related to the probability of suppression. That is, as the state increases the probability of suppression increases and as the state decreases the probability of suppression decreases. The observation equation describes how the observations relate to the unobservable state process. The objective is, thus, to estimate the brain's burst suppression state, burst suppression probability, and their associated confidence intervals.

One may assume that in each interval $\Delta$ there can be at most $n$ suppression events. Let $b_i$ be the number of suppression events in $i\Delta$. Further assume the observation model is described by the binomial probability mass function as:

$$f(b_i \mid x_i, n) = \binom{n}{b_i} p_i^{b_i} (1 - p_i)^{n - b_i}, \qquad (1);$$

where $p_i$ defined by the logistic function:

$$p_i = \frac{\exp(x_i)}{1 + \exp(x_i)} \qquad (2);$$

is the BSP and $x_i$ is the brain's burst suppression state at time $i$. In other words, $p_i$ is the instantaneous probability of burst suppression. The logistic function links the brain's burst suppression state to the probability of a suppression event and insures that $p_i$ remains between 0 and 1 as $x_i$ ranges across all real numbers.

The state model may be defined as a random walk:

$$x_i = x_{i-1} + \epsilon_i, \qquad (3);$$

where the $\varepsilon_i$ are independent Gaussian random variables with mean 0 and variance $\sigma_\epsilon^2$. This definition of the state provides a stochastic continuity constraint, which insures that the states and, hence, the BSPs that are close in time are close in value. The parameter $\sigma_\epsilon^2$ governs how rapidly the BSP can change; the larger (smaller) the value of $\sigma_\epsilon^2$ the more rapidly (slowly) the state and the BSP can change.

To present the estimation algorithm one may take b = ($b_1$, $b_2$,..., $b_I$) and x = ($x_1$, $x_2$,..., $x_I$). Based on the random walk defined in (3), the joint probability density of the state process is:

$$f(x \mid \sigma_\epsilon^2, x_0) =$$

$$\left( \frac{1}{2\pi\sigma_\epsilon^2} \right)^{\frac{I}{2}} \exp\left( \frac{-1}{2\sigma_\epsilon^2} \sum_{i=1}^{I} (x_i - x_{i-1})^2 \right) \tag{4};$$

and the joint probability density of the observed suppression events is:

$$F(b \mid x, n) = \prod_{i=1}^{I} \binom{n}{b_i} p_i^{b_i} (1 - p_i)^{n - b_i} \tag{5}.$$

An objective is to estimate using maximum likelihood (ML) the state process x and the parameters $\sigma_\epsilon^2$ and $x_0$, where the initial state $x_0$ is treated as a parameter. Once these estimates are obtained, the BSP can be readily computed with its confidence intervals.

To compute the ML estimates of the parameters we can use an approximate expectation maximization (EM) algorithm for point processes and binary time series. The EM algorithm is a method simultaneously models parameters and an unobservable state process by maximizing the expectation of the complete data log likelihood, doing so by iterating between two steps. In the expectation step, it computes the expected value of the complete data log likelihood given an estimate of the parameters from the previous iteration. In the following maximization step, it computes the parameters that maximize it.

The complete data likelihood is:

$$f(b, x \mid n, \sigma_\epsilon^2, x_0) = f(b, x \mid n) f(x \mid \sigma_\epsilon^2, x_0) \tag{6}.$$

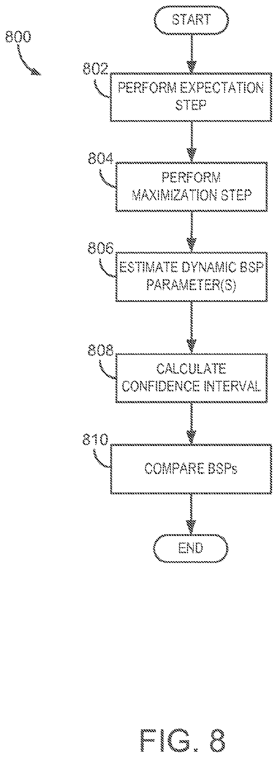

Referring to Fig. 8, one exemplary EM algorithm 800 will be described. At process block 802, an expectation step is performed. The expectation step at process 802 is actually representative of an iterative process. The first iteration starts at a default or otherwise selected position. At iteration $l + 1$, the expected value is computed of the complete data log likelihood given the data b and the estimates T($l$) and $x_0(l)$ of the parameters from iteration $l$ using the following:

$$Q(\sigma_\epsilon^{2}(\ell+1), x_0(\ell+1) \mid \sigma_\epsilon^{2}(\ell), x_0^\ell)$$

$$= E[\log(f(b, x \mid n, \sigma_\epsilon^2, x_0)) \mid\mid b, \sigma_\epsilon^{(2\ell)}, x_0^{(\ell)}$$

$$= E[\Sigma_{i=1}^{I} \log \frac{n!}{b_i!(n-b_i)!}$$

$$+ b_i x_i - \log(1 + e^{x_i}) \mid\mid b, \sigma_\epsilon^{(\ell)2}, x_0^{(\ell)}]$$

$$+ E[\Sigma_{i=1}^{I} -\frac{1}{2\sigma_\epsilon^2}(x_i - x_{i-1})^2$$

$$-\frac{I+1}{2}\log(2\pi) - \frac{I+1}{2}\log(\sigma_\epsilon^2)$$

$$-\frac{x_0^2}{\sigma_\epsilon^2} \mid\mid b, \sigma_\epsilon^{2(\ell)}, x_0^{(\ell)}] \tag{7}.$$

Expanding the right side of eqn. (7) illustrates the need to estimate three quantities for $i = 1,..., I$. The expectation of the state variable conditioned on the data up to time $I$:

$$x_i \mid I \equiv E[x_i \mid\mid b, \sigma_e^{2(\ell)}, x_0^{(\ell)}] \tag{8};$$

and the covariances of the state variable conditioned on the data up to time $I$:

$$W_{i,i \mid I} \equiv E[x_i^2 \mid\mid b, \sigma_e^{2(\ell)}, x_0^{(\ell)}] \tag{9};$$

and

$$W_{i,i-1 \mid I} \equiv E[x_i x_{i-1} \mid\mid b, \sigma_e^{2(\ell)}, x_0^{(\ell)}]. \tag{10}.$$

In order to compute these quantities efficiently, the expectation step can be divided into three parts. First, the estimates of $x_{i|i}$ and $\sigma_{i|i}^2$ are computed using the forward binary or, in accordance with one aspect of the invention, a "BSP" filter, which is a specific implementation of a binary filter. Second, the backward fixed interval smoothing (FIS) algorithm is used to compute $x_{i|I}$ and $\sigma_{i|I}^2$. Finally, state space covariance algorithm is used to compute the covariances $W_{i|I}$ and $W_{i,i-1|I}$.

With respect to the binary filter, given the parameters estimates from iteration $I$, this step estimates $x_{i|i}$ and $\sigma_{i|i}^2$. This means that the step will estimate the state and the variance at $i$ looking at data from the start of the experiment up to $i$ using a non linear recursive forward filter algorithm.

A one step prediction mean and variance are given by:

$$x_{i\,|\,i-1} = x_{i-1\,|\,i-1}, \qquad (11);$$

and

$$\sigma^2_{i\,|\,i-1} = \sigma^2_{i-1\,|\,i-1} + \sigma^2_e(\ell). \qquad (12).$$

The posterior mode and variance are given by:

$$x_{i\,|\,i} = x_{i\,|\,i-1} + \sigma^2_{i\,|\,i-1}(b_i - np_{i\,|\,i}), \qquad (13);$$

and

$$\sigma^2_{i\,|\,i} = [(\sigma^2_{i\,|\,i-1})^{-1} + np_{i\,|\,i}(1 - p_{i\,|\,i})]^{-1}. \qquad (14).$$

The initial conditions are $x_{0|0} = x_0(l)$ and $\sigma_{0|0}^2 = \sigma_e^2(l)$. $p_{i|i}$ corresponds to the mode of the posterior distribution. This filter is non-linear because $x_{i|i}$ appears on both sides of (13). It could be calculated recursively using Newton's method, however, when the width of $\Delta$ is very small, adjacent states are very close, and we can replace the term $p_{i|i}$ in equation (13) by $p_{i-1|i-1}$.
With respect to the fixed interval smoother, the posterior mode estimates from the forward filter are used by the FIS to yield the estimates $x_{i|I}$ for $i = I - 1,..., 1$. This means that the estimate at time $i$ is conditioned on all the data up to time $I$. It is a linear filter and the final estimate of the state will thus be a Gaussian distributed variable with mean $x_{i|I}$ and variance $\sigma_{i|I}^2$. The FIS is:

$$x_{i\,|\,I} = x_{i\,|\,i} + A_i(x_{i+1\,|\,I} - x_{i+1\,|\,i}), \qquad (15);$$

$$A_i = \sigma^2_{i\,|\,i}(\sigma^2_{i+1\,|\,i})^{-1} \qquad (16);$$

and

$$\sigma^2_{i\,|\,I} = \sigma^2_{i\,|\,i} + A_i^2(\sigma^2_{i+1\,|\,I} - \sigma^2_{i+1\,|\,i}) \qquad (17).$$

The initial conditions are $x_{i|I}$ and $\sigma_{i|I}^2$ previously estimated in the filter algorithm.
With respect to the state space covariance algorithm, $\sigma_{i,j|I}$ can be derived as follows:

$$\sigma_{i,j\,|\,I} = A_i\sigma_{i+1,j\,|\,I}, \qquad (18);$$

where $1 \le i \le j \le I$. The covariances are thus given by:

$$W_{i,i-1\,|\,I} = \sigma_{i,i-1\,|\,I} + x_{i\,|\,I}x_{i-1\,|\,I} \qquad (19);$$

and

$$W_{i,i \mid I} = \sigma^2_{i \mid I} + x^2_{i \mid I}. \qquad (20).$$

Referring again to Fig. 8, the process 800 continues at process block 804 with the performance of a maximization step. In this context, let $\tau = 1/\sigma_\varepsilon^2$. One may assume that r has a gamma prior density defined as:

$$f(\tau \mid \alpha, \beta) = \frac{\beta^\alpha}{\Gamma(\alpha)} (\tau)^{\alpha - 1} \exp(-\beta\tau). \qquad (21).$$

The complete data log likelihood is maximized with respect to $\tau$ using the gamma prior density for $\tau$ in (21) and then the log posterior is maximized with respect to $\tau$. The expected value of the complete data log likelihood serves as the likelihood in the expression for the posterior.

The log posterior density of $\tau$ is proportional to:

$$\log(f(\tau \mid \alpha, \beta)) + E[\log[f(b, x \mid n, \tau, x_0)] \mid\mid b, \tau^{(\ell)}, x_0^{(\ell)}$$

$$= (\alpha - 1)\log(\tau) - \beta\tau + \alpha \log)(\beta) - \log(\Gamma(\alpha)) \qquad (22).$$

$$+ \frac{I}{2}\log(\tau) - \frac{\tau}{2}E[\Sigma_{i=1}^{I}(x_i - x_{i-1})^2 + 2x_0^2 \mid\mid b, \tau^\ell, x_0^\ell]$$

By maximizing (22) with respect to $\tau(l+1)$ one obtains:

$$\tau(\ell + 1)$$

$$= [I + 2\alpha - 2][2(\Sigma_{i=2}^{N} W_{i \mid I} - \Sigma_{i=2}^{N} W_{i-1, i \mid I}) \qquad (23).$$

$$+ \frac{3}{2}W_{1 \mid I} - W_{I \mid I} + 2\beta]^{-1}$$

By maximizing (23) with respect to $x_0(l+1)$ one obtains:

$$x_0(\ell + 1) = \frac{1}{2}x_{1 \mid I} \qquad (24).$$

The maximum likelihood estimates of $\tau$ or equivalently $\sigma_\varepsilon^2$ and $x_0$ are respectively $\tau(\infty) = \sigma_\varepsilon^{-2}(\infty)$ and $x_0(\infty)$.

Thereafter, at process block 806, dynamic BSP estimated and, at process block 808, the above-described confidence intervals are calculated. Specifically, the algorithm iterates between the expectation and maximization steps until convergence. The fixed-interval smoothing algorithm evaluated at the ML estimates $x_0$ and $\sigma_\varepsilon^2$ together with the logistic equation (2) give us the probability of a suppression at time i for $i = 1,...,I$. Through a change of variable, the probability density function may be computed which corresponds to the BSP estimate using:

$$f(p \mid x_{i \mid I}, \sigma^2_{i \mid I}) = f(x \mid x_{i \mid I}, \sigma^2_{i \mid I}) \left| \frac{dx}{dp} \right|$$

$$= [(2\pi\sigma^2_{i \mid I})^{\frac{1}{2}} p(1 - p)^{-1}$$

$$\times \exp(- \frac{1}{2\sigma^2_{i \mid I}} (\log[p(1 - p)^{-1}] - x_{i \mid I})^2) ]$$

(25).

The confidence intervals are obtained by computing the cumulative density of equation (25) and identifying the 2.5th and 97.5th percentiles.

At process block 810, a comparison of BSPs at different times can be performed. Because the logistic transformation that relates the state to the BSP is monotonic, the probability that the BSP at time $i$ is greater than the BSP at time $j$ is obtained by computing the corresponding probabilities of the states. This is done through a Monte Carlo approach. Using the covariance algorithm, for times $i$ and $j$ such that $1 \leq i < j,$ the covariance between the augmented state space trials is given by:

$$W_{i,j \mid I} = \prod_{k = i}^{j - 1} A_k W_{j,j \mid I}$$

(26).

Next, one can then draw M samples from the Gaussian distribution with mean $\begin{bmatrix} x_{i \mid I} \\ x_{j \mid I} \end{bmatrix}$ and covariance

matrix $\begin{bmatrix} W_{i,i \mid I} & W_{i,j \mid I} \\ W_{j,i \mid I} & W_{j,j \mid I} \end{bmatrix}$ and count the number G of instances in which the relevant probability is the estimate of $Pr(x_{i \mid I} > x_{j \mid I})$, such that:

$$P(x_{i \mid I} > x_{j \mid I}) = \frac{G}{M}.$$

(27).

As described above, the present invention allows the estimation of BSP on a second scale and allows formal statistical comparisons of burst activity at different time points by constructing the confidence intervals of the estimates. The BSP algorithm estimates the joint distribution of the state process, whereby $Pr(x_i > x_j)$ can be evaluated for any $0 \leq i < j \leq N$. This is equivalent to the probability that the BSP at time $i$ is greater than the BSP at time $j$ because the transformation between the state variable $x_i$ and the BSP $p_i$ is monotonic. The state-space model of the present invention suggests a more principled and informative way to analyze and control this key EEG brain state. Consequently, formal comparisons can be made not only between pre-selected time points but across entire experiments between them and significant changes that occur can be tracked. In one aspect, it may be used for tracking brain states during a medical procedure, such as the administration of an anesthetic or drug, or to assess the progression of disease states, such as a medical coma. In another aspect, it may be used to reveal the dynamical structure of different brain patterns.

*Closed-loop monitor/control using burst suppression*

The above-described, the BSP algorithm has wide applicability, for example, in both traditional, clinician-controlled environments, but also when utilizing closed-loop control and drug delivery systems. That is, in one embodiment of the present invention, a closed-loop anesthesia delivery and control system is provided, which may utilize the afore-described BSP algorithm.

The following description of a the closed-loop monitoring and drug delivery control system has wide clinical application. However, for exemplary purposes, the following description will be made with respect to the clinical application of automatic control of medical coma using closed-loop regulation of an anesthetic drug to maintain a specified level of medical coma in terms of a specified level of burst suppression. In this application, the measured level of burst suppression is used as a feedback signal by which the anesthetic infusion is adjusted in a continuous, optimal manner.

While the maintenance of burst suppression is not an objective for general anesthesia, it is a means for providing a stable state of medical coma to aid patients who are recovering from brain injury. For most, drugs are administered at a specified rate, clinical examinations are conducted intermittently, and no continuous EEG tracking of brain state is performed. A common concern with this approach is the eventual overdosing of the drug and the subsequent life-threatening sequelae of these overdose syndromes. It is ironic that the medical coma is a brain state targeted for therapeutic purposes, yet the state of the brain is not generally continuously monitored and controlled. Simulation results provide compelling evidence that maintenance of the brain in a medical coma at a precise level of burst suppression is highly feasible.

In one clinical application, the control target may be to achieve and maintain a target BSR corresponding to medical coma, thereby maintaining an equilibrium effect-site concentration. To design a suitable but not overly-complex model, it may be assumed that, due to the time lag between central compartment drug infusion and effect-site concentration increase, there must be at least two compartments to accurately model the evolution of the drug concentrations. Such a simplified two-compartment model is composed of the central plasma compartment and the auxiliary effect-site compartment connected by a first-order transfer process, ignoring any other peripheral drug distribution compartments.

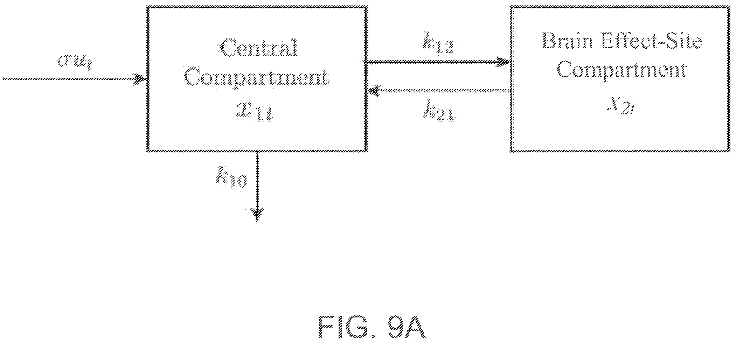

The pharmacokinetic model in Fig. 9A can be described by the secondorder linear differential equation system:

$$\frac{dx(t)}{dt} = Ax(t) + I(t) \tag{28};$$

where:

$$x(t) = \begin{bmatrix} x_{1t} \\ x_{2t} \end{bmatrix} \tag{29a};$$

$$A = \begin{bmatrix} -(k_{12} + k_{10}) & k_{21} \\ k_{12} & -k_{21} \end{bmatrix} \tag{29b};$$

$$I(t) = \begin{bmatrix} \sigma u_t \\ 0 \end{bmatrix}. \tag{29c};$$

and

for an infusion rate of $u_t$, in units of $\mu$g/s, scaled by a factor of $\sigma$. The kinetic rate constants $k_{12}$ and $k_{21}$ govern the drug flow between compartments, while the rate constant $k_{10}$ determines the rate of the drug's clearance from the central compartment. The slow peripheral compartment and fast peripheral compartment, common in many population models, are not included. Once the parameters of this model have been estimated for a subject- and drug-specific model, control simulations can be performed to examine the behavior of the system.

To relate the concentration of the drug in the effect compartment to the propensity of the brain to be in a state of burst suppression, equation (2) may be modified to define the BSP at a given time $t$ as:

$$p_t = \frac{1 - e^{-x_{2t}}}{1 + e^{-x_{2t}}}.$$  (30).

Equation (30) maps the drug concentration in the brain's effect compartment, a number on the interval $[0,\infty)$, to a BSP value on $(0,1)$. BSP is a more appropriate term than the BSR defined by Vijn and Sneyd or Rampil and Laster, as it shows explicitly that the index is a number between 0 and 1. Moreover, the BSP leads to a more principled method to relate the EEG to the effect-sitecompartment concentration for this problem.

The model in (28)-(30) would be a sufficient starting point from which to design a deterministic controller for a closed-loop control system, provided one could observe either $x_{2t}$ or $p_t$ directly. However, this is not the case since only the EEG signal, which is a stochastic process, can be observed. To give a more precise mathematical formulation of the control problem, a stochastic model that relates the EEG to $x_{2t}$ and $p_t$ can be defined. To do so, a binary filter algorithm can be implemented, which allows the computation of a dynamic estimate of BSP, $\hat{p}_{t|t}$, from a thresholded EEG signal and input an error signal in real-time to a system controller.

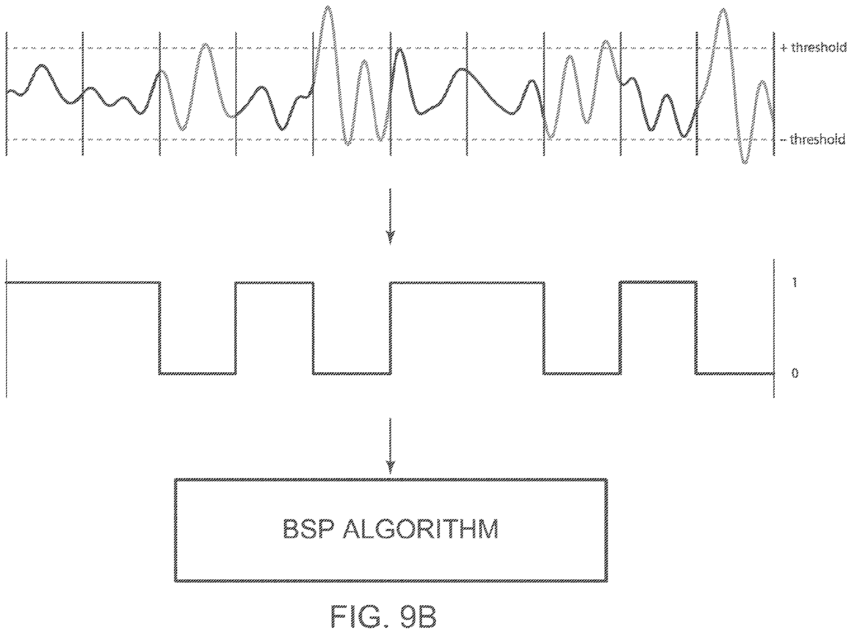

Turning now to Fig. 9B, an example of how a binary filter may process EEG signals is provided. Thresholding is achieved by differencing the EEG and defining a burst period interval, for example, 100-ms in duration, for which the absolute value of the differenced EEG signal (digitized at any desired frequency, such as, 641.03 Hz) is greater than a threshold (say 15 $\mu$V) at any point of its duration. Similarly, an interval is defined as a suppression period if the absolute value of the differenced EEG signal does not exceed the threshold (say 15 $\mu$V) throughout the interval. Therefore, the thresholded EEG signal may be written as a binary time series defined on any interval $t$:

$$n_t = n_t \begin{cases} 0 \text{ if the interval is a burst interval} \\ 1 \text{ if the interval is a suppression interval} \end{cases}$$  (31).

It may be assumed that the BSP, $p_t$, is the probability that $n_t$ is in the suppressed state. It follows that on any interval $t$, $n_t$ is a Bernoulli random variable defined by:

$$\Pr(n_t) = p_t^{n_t} (1 - p_t)^{1 - n_t}.$$  (32).

To complete the stochastic model of the EEG and to estimate $pt$ in real-time from $n_t$, one may defined define:

$$z_t = \log(x_{2t})$$  (33);

and assume that it obeys the random walk model:

$$z_t = z_{t-1} + v_t$$  (34);

modified from equation (3), where $v_t$ is independent Gaussian noise with zero mean and variance $\sigma_v^2$. Equation (34) is a stochastic continuity constraint that ensures that the updated value of the BSP will be close to the immediately preceding value. The degree of stochastic continuity is governed by $\sigma_v^2$. The larger (smaller) the value of $\sigma_v^2$, the greater (lesser) the degree of allowable change in the BSP between adjacent intervals. An estimate for $\sigma_v^2$ may be taken to be:

$$\hat{\sigma}_v^2 = \frac{1}{T} \sum_{t=2}^{T} (z_t - z_{t-1})^2$$  (35);

where $z_t$ denotes the Vijn and Sneyd BSR data transformed using equations (30) and (33). For both models, one may take $\sigma_v{}^2 = 10^{-5}$, although other values are possible.

It follows from Chemali JJ, Wong KFK, Solt K, Brown EN, "A state-space model of the burst suppression ratio," IEEE EMBC Sep 2011, Boston, MA, that $pt$ may be estimated in real-time from the time series of $n_t$ by using a binary filter defined for this model as

$$z_{t\,|\,t-1} = z_{t-1\,|\,t-1} \tag{36a};$$

$$\sigma^2_{t|t-1} = \sigma^2_{t-1|t-1} + \sigma^2_v \tag{36b};$$

$$z_{t\,|\,t} = z_{t\,|\,t-1} + \frac{\sigma^2_{t\,|\,t-1} c_t}{p_t(1-p_t)}(n_t - p_t) \tag{36c};$$

$$\sigma^2_{t\,|\,t} = \left[ \frac{1}{\sigma^2_{t\,|\,t-1}} + \frac{c^2_t}{p_t(1-p_t)} \right]^{-1} \tag{36d};$$

where:

$$c_t = \frac{\partial p_t}{\partial x_{2t}}\frac{\partial x_{2t}}{\partial z_t} = \frac{x_{2t}e^{x_{2t}}}{e^{x_{2t}}+1}(1-p_t). \tag{36e}.$$

Note the modification of equations (11) through (14). Equations (36c) and (36d) are implicit functions in $z_t$. To solve, an implementation of Newton's method is used, with stopping criteria:

$$\left| z^{(n)}_{t\,|\,t} - z^{(n-1)}_{t\,|\,t} \right| < 10^{-9} \tag{37a};$$

and

$$\left| f\left( z^{(n)}_{t\,|\,t} \right) \right| < 10^{-6} \tag{37b}.$$

The function for which a root is desired is:

$$f(z_{t \mid t}) = -\frac{z_{t \mid t} - z_{t \mid t-1}}{\sigma^2_{t \mid t-1}} + \frac{c_t(n_t - p_t)}{p_t(1 - p_t)} \qquad (38);$$

and due to the small time interval between successive $n_t$ values, $z_{t-1 \mid t-1}$ is an appropriate initial estimate for $z_{t \mid t}$ to begin the Newton's method. To reduce computation, the expected value of $f'(y_{t \mid t})$ may be used, since $p_t = E(n_t)$:

$$E[f'(z_{t \mid t})] = -\left[\frac{1}{\sigma^2_{t \mid t-1}} + \frac{c_t^2}{p_t(1 - p_t)}\right]. \qquad (39).$$

Thus, each successive estimate of the root, $Z_{t \mid t}^{(n+1)}$, may be calculated by a local Fisher's scoring algorithm:

$$z_{t \mid t}^{(n+1)} = z_{t \mid t}^{(n)} - E\left[f'\left(z_{t \mid t}^{(n)}\right)\right]^{-1} f\left(z_{t \mid t}^{(n)}\right). \qquad (40).$$

Once the stopping criteria are satisfied (which may occur in less than 10 iterations), $z_{t \mid t}$ and $\sigma_{t \mid t}$ are taken to be their estimates from the final iteration. By applying this binary filter to the time series $n_t$, one can compute an estimate of $p_t$ and use it in real-time to compute an error signal for input to a controller.

The stochastic BSP model and binary filter algorithm of the present invention give a principled near-optimal procedure for estimating BSP from the thresholded EEG. Even though $\hat{p}_{t \mid t}$ is a stochastic signal, because adjacent estimates are separated by milliseconds, this signal is sufficiently smooth and may be used as an input to a deterministic control system.

To simulate a stochastic control problem, the output of the infusion-driven differential equation system is transformed to $pt$ and used at each time interval dt to generate a vector of binary values of length (Fs x dt), where $Fs$ denotes the binary filter's input frequency of $n_t$. In one embodiment, a binary filter input frequency of 200 Hz may be used, although other frequencies are possible, with a time interval of 1 second. The binary filter iterates through these integers and generates an updated estimate to the propensity of burst suppression, $\hat{p}_{t \mid t}$.

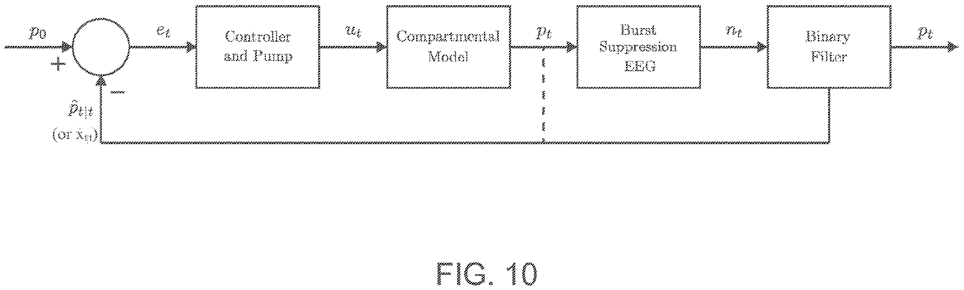

This dynamic estimation of the BSP can be used as negative feedback to generate the error signal that inputs to a proportional-integral (PI) controller. The efficacy of the controller can then be tested in the context of a stochastic feedback signal. Referring to Fig. 10, a closed-loop control can be implemented using a PI scheme, where the control input is calculated as:

$$u_t = K_p e_t + K_i \sum_t e_t. \qquad (41).$$

The signal $et$ is the error defined by:

$$e_t = \bar{x}_2 - x_{t \mid t}, \qquad (42)$$

where $\bar{x}_2$ corresponds to the target BSP. In this scheme, the current state estimation, $\hat{p}_{t \mid t}$, is subtracted from the target BSP, $p_0$, to generate the error signal, $e_t$. This error is processed by the controller, which appropriately adjusts the pump's infusion rate, $u_t$, to drive the compartmental pharmacokinetics model. The system of differential equations that makes up this underlying system is evaluated numerically to yield $x_{2t}$, which is transformed to $p_t$. In the deterministic simulation scenarios, this $p_t$ is taken as the current state estimation, with or without added Gaussian error. In the stochastic simulation

scenario, this $p_t$ is used to generate a sequence of Bernoulli binary integers, $n_t$, which are processed by the binary or BSP filter to yield a principled state estimation, $\hat{p}_{t|t}$.

The feedback signal $X_{t|t}$ is the output of the binary filter. The gains $K_p$ and $K_i$ may be chosen in order to achieve suitable performance in terms of the closed loop step-response of the deterministic system, that is, when $X_{t|t} = X_t$. Specifically, it may be advantageous to seek gains that ensure fast rise-time while minimizing overshoot, a potentially undesirable feature in anesthetic induction. A performance specification of this type can be achieved by ensuring that the dominant poles of the closed loop system lie within a prescribed region of the complex plane. In order to complete this procedure, one may use the continuous-time analog of (41) to obtain the open-loop system transfer function:

$$H(s) = \frac{X_2(s)}{E(s)} = \frac{(sK_p + K_i)\sigma k_{12}}{s(s + k_{10})(s + k_{21})}. \tag{43}$$

Here, $X_2(s)$ and $E(s)$ are the Laplace transforms of $x_2t$ and $e_t$, respectively. It follows from (43) that the poles of the closed-loop system are the solutions of the characteristic equation:

$$1 + H(s) = 0. \tag{44}$$

From (43) and (44) $K_i$ and $K_p$ may be selected to affect the location of the closed-loop poles within the s-plane. Given a desire to avoid unnecessary overshoot, one may aim for the fastest dynamics for which the closed-loop poles lie entirely on the real line. In the case of the closed-loop system, it amounts to real and repeated roots of equation (44).

To simplify the analysis, the ratio $K_i$ and $K_p$ may be set to:

$$\frac{K_i}{K_p} = \min\{k_{10}, k_{21}\}, \tag{45}$$

thereby inducing a pole-zero cancellation in (43), yielding:

$$H(s) = \frac{\tilde{X}_2(s)}{E(s)} = \frac{\sigma k_{12} K_p}{s(s + \max\{k_{10}, k_{21}\})}. \tag{46}$$

Substituting this simplified open-loop transfer function into (44), the poles of the closed-loop system are given by the solutions of:

$$s^2 + \max\{k_{10}, k_{21}\}s + \sigma k_{12} K_p = 0. \tag{47}$$

Given the design goal of repeated real roots, one may choose $K_i$ and $K_p$ to satisfy:

$$\sqrt{\max\{k_{10}, k_{21}\}^2 - 4\sigma k_{12} K_p} = 0. \tag{48}$$

Thus, it follows that the optimal PI controller gains for achieving desired specifications are:

$$K_p = \frac{\max\{k_{10}, k_{21}\}^2}{4\sigma k_{12}} \qquad (49);$$

and

$$K_i = K_p \min\{k_{10}, k_{21}\}. \qquad (50).$$

Accordingly, the control gains can be computed in a manner that is specific to the underlying system parameters.

The controller architecture described above is highly robust to parametric uncertainty. This robustness may be characterized in terms of stability margins. In particular, one may assume that the 'true' parameters $k_{10}$, $k_{21}$ deviate from the model by multiplicative factors $\sigma_{10}$ and $\sigma_{21}$, respectively. It follows from (43), (49), and (50) that the open loop transfer function is given by:

$$H(s) = \frac{X_2(s)}{E(s)} = \frac{K_p(s + \min\{k_{10}, k_{21}\}\sigma k_{12}}{s(s + \sigma_{10}k_{10})(s + \sigma_{21}k_{21})}. \qquad (51).$$

Using a standard root-locus argument, it can be verified that the poles of the corresponding closed-loop system are stable for all $K_p > 0$ if the following condition holds:

$$\min\{k_{10}, k_{21}\} < \sigma_{10}k_{10} + \sigma_{21}k_{21}. \qquad (52);$$

where a conservative sufficient condition for (52) is:

$$\sigma_{10} + \sigma_{21} > 1, \qquad (53);$$

which constitutes a generous tradeoff relationship in parametric uncertainty. For instance, the parameters $k_{10}$ and $k_{21}$ would require misidentification by at least 50% in order to compromise the closed-loop stability. Note that this criterion ensures stability for all $K_p > 0$, which includes the choice of $K_p$ as in equation (49). Moreover, it ensures that arbitrary uncertainty in $k_{12}$ or $\sigma$ will not destabilize the system (since such uncertainty enters multiplicatively in $K_p$).

By designing a closed-loop system to have real poles, generous phase margins are ensured, that is, sensitivity to uncertain time-delays. Since (49) and (20) in this approach leads to real-valued poles, these margins may be on the order of 75 degrees. Given that the open-loop system dynamics are relatively slow, this amounts to a tolerance of time delays on the order of minutes.

From (36c) the estimate $x_{t|t}$ may be represented as:

$$x_{t|t} = x_t w_t, \qquad (54);$$

where $w_t$ is a lognormal random variable generated from a Gaussian of zero mean and variance $\sigma_{t|t}^2$. From (14), conceptually this can be viewed as a parametric uncertainty for the feedback gains $K_i$, $K_p$. However, as shown above, the PI design in (22) and (23) has large stability margins - indeed, $K_p$ has infinite gain margin.

Moreover, it is known from the binary filter algorithm that $\sigma_{t|t}^2$ is bounded. It thus follows that the trajectories of the stochastic closed-loop system have finite variance about $\bar{x}_2$, a property which has been verified in the Monte Carlo simulation pursued herein.

The above-described systems and methods may be further understood by way of examples. These examples are offered for illustrative purposes only, and are not intended to limit the scope of the present invention in any way. Indeed, various modifications of the invention in addition to those shown and described herein will become apparent to those skilled in the art from the foregoing description and the following examples and fall within the scope of the appended claims. For example, specific examples of brain states, medical conditions, and so on, in association with specific drugs and procedures are provided, although it will be appreciated that other drugs, doses, conditions and procedures may also be considered within the scope of the present invention. Likewise, process parameters are recited (for example, signal processing,) that may be altered or varied based on variables such as signal duration, intensity, incidence rate, and so forth.

EXAMPLE I

*Physostigmine effect on stable burst suppression*

The following description is with respect to an analysis of a rat under general anesthesia-induced burst suppression The BSP is compared to the BSR in order to illustrate its benefits. For the experiments described herein, signals were first band pass filtered between 5 and 30Hz. The filtered signals were thresholded and suppression segments less than 500 milliseconds in duration were switched to 1. The binary series was then provided as an input to the BSP algorithm.

The BSP algorithm was evaluated on a rat EEG signal recorded to test whether physostigmine, a cholinergic agonist hypothesized to increase arousal, causes the burst suppression pattern observed during deep anesthesia to switch into continuous activity (associated with increased arousal). In this experiment, it is advantageous to know whether physostigmine, and not saline (control), induces a shift from burst suppression (deep anesthesia) to a delta wave pattern (lighter anesthesia). In case it does, it is desirable characterize the temporal dynamics of that change.

A rat pre-implanted with extradural EEG electrodes was deeply anesthetized with 2% isoflurane. After stabilization of the inhaled concentration, the EEG signal, was recorded for 10 minutes prior to a saline intravenous injection (control). Six minutes later, physostigmine was injected and the recording was maintained for an additional thirty minutes. The total observation interval was of 40 minutes, where the EEG signal was sampled at 512 Hz. The choice of $\Delta = 512$ corresponds to a resolution of one second. Both the BSR and the BSP were computed in one second epochs to maximize the detection of dynamics.

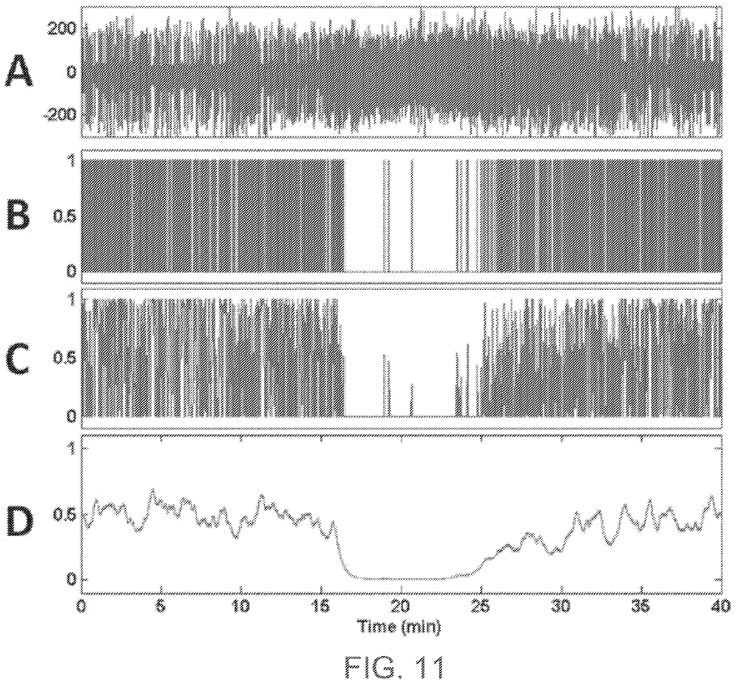

Figs. 11A-D shows the complete raw signal sampled at 512 Hz (Fig. 11A), the corresponding binary signal (Fig. 11B), the BSR estimates (Fig. 11C) and the BSP estimate (Fig. 11D). At this resolution, the BSR is a very noisy signal that cannot be used for interpretation without further processing. Although there is a clear drop in the BSR at the injection of physostigmine, there is no principled way to measure statistical significance of the drop. Typically, in these experiments, the protocol is repeated in several rats in order to make inferences about the drop.

In contrast, the BSP, using the same resolution, gives a smooth estimate. It is easily seen that the brain state is globally at a stable burst suppression state (BSP is around 0.5). At the injection of physostigmine, an arousal drug, the rat promptly comes out of suppression (BSP is 0) for around 10 minutes at which point the suppression segments reappear and slowly increase to reach back the baseline probability. Because of the fine resolution, the natural fluctuations of the BSP around its mean value are also discerned. For instance, for the BSP between minute 4 and 7, the BSP increases steeply over one minute and then decreases slowly over a few minutes. This means that a long suppression period was then followed by a pattern where burst periods were moderately longer than the suppression periods.

Although the saline injection at minute 16 does not induce any change in dynamics, it is interesting to note the difference in estimates around minute 20 between the BSR and BSP. The BSR is a memory-less measure, which computes the fraction of 1s in each bin without taking into consideration that the EEG signal is a dynamic time-series. Therefore, at minute 18 and 22, the BSR peaks at 0.5 and 0.2 respectively although the overall state clearly does not justify these estimates. In contrast, the BSP estimate consistently remains very close to 0 for this segment. Furthermore, the computed confidence intervals of the estimates allow formal statistical inferences to be made.

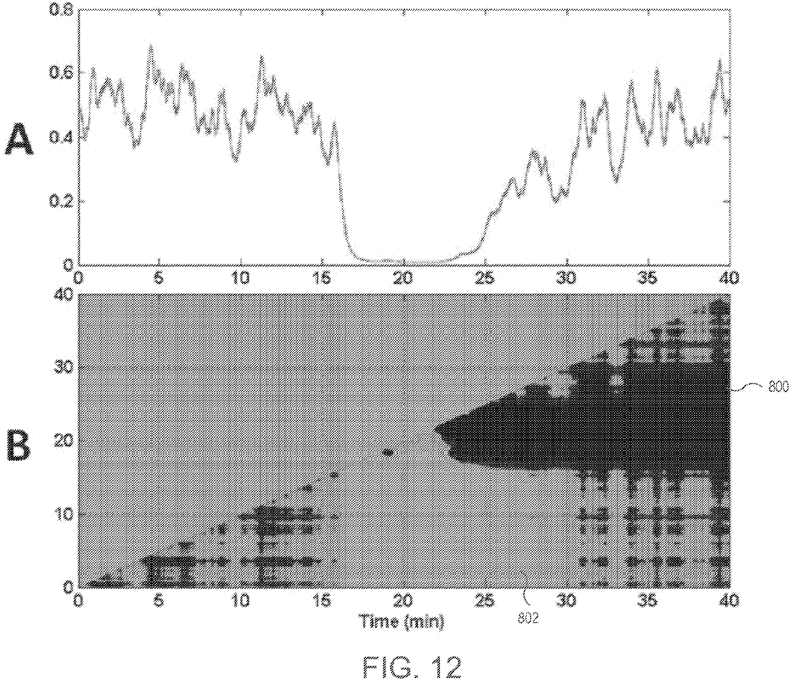

Fig. 12A which shows a BSP estimate waveform of a Sprague-Dawley rat with confidence intervals computed in one second epochs.

Fig. 12B is a point-by-point triangular comparison matrix, showing how every point along the horizontal time axis is compared to all preceding points in time. Evaluating $Pr(x_i > x_j)$ for the waveform of Fig. 12A, where $x_j$, the y-axis, corresponds to the BSP at time $j$ and $x_i$, the x-axis, corresponds to the BSP at time for I, darkercolored areas 800 illustrate that the corresponding value on the horizontal axis $x_i$ is greater than the corresponding value on the vertical axis $x_j$ with a probability of 0.95. Similarly, lighter-colored areas 802 illustrate that the corresponding value on the horizontal axis x; is smaller than the corresponding value on the vertical axis $x_j$ with a probability of 0.95. In other words, a light-colored entry corresponds to $Pr(x_i < x_j) = 0.95$ and a dark entry corresponds to $Pr(x_i > x_j) = 0.95$ where $i$ is the index across the horizontal axis and $j$ is the index across the vertical axis.

It is appreciated from the pattern of light-colored and dark-colored dots in the first 15 minutes and last 10 minutes, that the fluctuations around the mean are not merely explained by a noisy signal, but reflect a significant dynamical structure. If they were not significant, all corresponding entries would have been gray. We also see, that the patterns pre- and post- saline injection are similar.

Moreover, although in this example the change is very dramatic and can be easily seen in the BSP plot, the point-to-point comparison matrix is a confirmation that after the injection of physostigmine at minute 16 until about minute 26 the BSP is significantly smaller than the BSP at all other time points. This abrupt drop in the BSP is expected because physostigmine is a cholinergic agonist that induces increased arousal.

This kind of comparison is advantageous in cases where changes in burst suppression are correlated with a condition of interest. For example, it can be correlated with the progression of a disease such as in subacute sclerosis panencephalitis. In these cases, a measure of significance is fundamental in guiding the diagnosis.

EXAMPLE II

*Burst suppression during hypothermia*

The above-described systems and methods have broad clinical applicability. One exemplary clinical application includes the ability to track of burst suppression during hypothermia. For example, consider the binary filter when used to assess the evolution of the hypothermia induced burst suppression level during a cardiac surgery of around three and a half hours.

The following description is with respect to an analysis of a patient under hypothermia-induced burst suppression. The BSP is compared to the BSR in order to illustrate its benefits. Signals were first band pass filtered between 5 and 30Hz. The filtered signals were then thresholded and suppression segments less than 500 milliseconds in duration were switched to 1. The binary series was then provided as an input to the BSP algorithm.

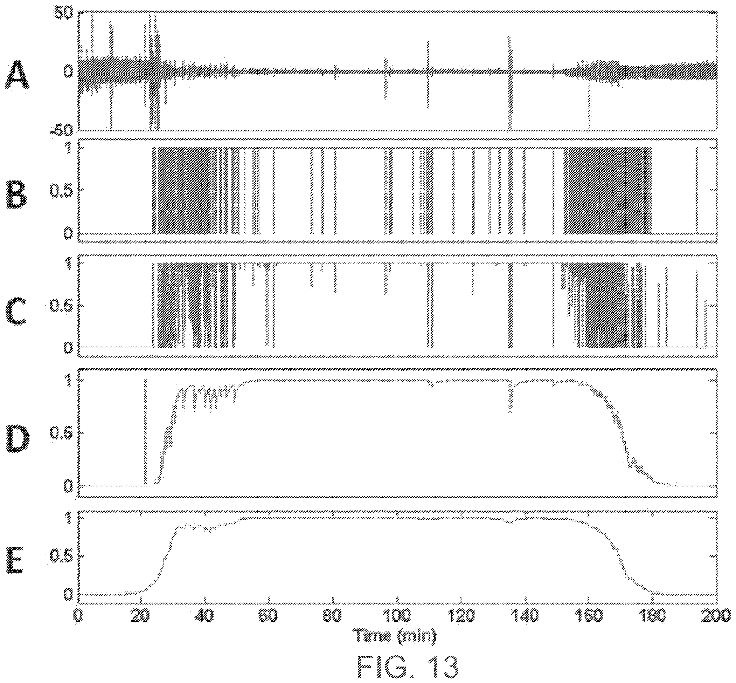

In this example, the EEG signal was recorded from a scalp electrode at the FP1 site referenced to the FZ electrode. The total observation interval was 208 minutes, and the EEG signal was sampled at 250 Hz. The choice of $\Delta = 250$ corresponds to a resolution of one second as in the previous case. Figs. 13A-E show the complete raw signal sampled at 250 Hz (Fig. 13A), the corresponding binary signal (Fig. 13B), the BSR estimate computed in one second epochs (Fig. 13C) the BSP estimate from the binary filter using the ML parameter estimates and (Fig. 13D) the BSP estimate from the smoothing algorithm (Fig. 13E).

The inputs to the binary filter consist of the binary signal, the initial value of the state process $x_0$, and noise variance $\sigma^2$, which determines how quickly the algorithm tracks the changes in the BSP. in several cases these parameters are readily approximated. The BSR in Fig. 13C shows that after an initial 25 minutes of continuous activity, the EEG enters a burst suppression mode that ceases around minute 50 ceases, turning into an almost isoelectric state. Symmetrically, at about the 150th minute, the burst suppression activity reappears for 25 minutes and the patient comes out of burst suppression. At this resolution, it is very difficult to visually track the level of burst suppression from the BSR.

The binary filter estimate in Fig. 13D is a smoother curve. The general trend of the pattern is very clear, where the increase and decrease are linear, taking around 10 minutes each. The patient remains for about 110 minutes at a stable, almost isoelectric brain state. After the initial increase the ripple in the BSP between minute 20 and 30 indicates that long suppression and long burst periods are present.

The BSP in Fig. 13E is the smoothest curve. It is almost symmetrical between cooling and rewarming. The increase and decrease is linear and takes around 10 minutes in both cases. The patient remains for about 110 minutes at a stable, almost isoelectric brain state.

To use the binary filter, it is possible to define as few as two parameters. Namely, the initial state $x_0$ and the noise variance $\sigma^2$ can be defined. In several practical cases, these parameters can be confined to a range of realistic values. The binary filter then successfully tracks in real-time the change in burst suppression. By contrast, the BSR is very noisy, and does not give a useful real-time estimate. This suggests that the forward filter is useful when continuous ongoing display of the EEG activity is of interest. Because it can be tracked at very fine resolution, and computes an estimate of the error, it enables the easy and reliable recognition of discrete events.

EXAMPLE III

*Burst suppression during propofol induction*

Another exemplary clinical application is the tracking of burst suppression during propofol bolus induction. Typically, in the operating room, a bolus dose of an anesthetic is rapidly administered to induce general anesthesia. It is often the case that the patient enters burst suppression within seconds and might remain in that state for several minutes. Since the efficiency of the drug depends on several empirical factors, it is relevant to monitor the level of suppression that is reached and its trajectory, which may help detect any anomaly, or tune the subsequent doses or levels of anesthesia.

The approach of the present invention was evaluated on a burst suppression pattern and its progression induced by a propofol bolus. The EEG was recorded from a scalp electrode at the FP1 site referenced to the FZ electrode of the standard electrode configuration. The total observation interval is of 17 minutes, where the EEG signal was sampled at 250 Hz. The choice of $\Delta$ = 250 corresponds to a resolution of one second as in the previous case.

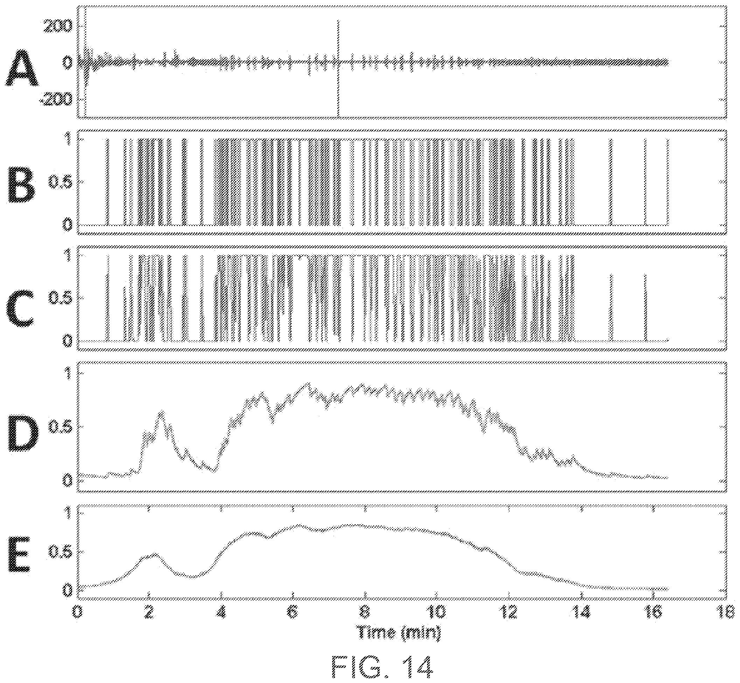

Figs. 14A-E show the complete raw signal sampled at 250 Hz (Fig. 14A), the corresponding binary signal (Fig. 14B), the BSR estimate computed in one second epochs (Fig. 14C) the BSP estimate from the binary filter using the ML parameter estimates and (Fig. 14D), and the BSP estimate from the smoothing algorithm (Fig. 14E). After the induction, the progression is not monotonous. The filter and smoother BSP climbs to around 0.5 and 0.6 respectively at minute 2, decreases to around 0.2 and 0.1, and then slowly increases to reach a BSP of around 0.7 decreases monotonically to 0. The burst suppression lasts for about 8 minutes, half of which is in an increasing trajectory and the other half in decreasing trajectory.

EXAMPLE IV

*Burst suppression control during propofol and etomidate*

Control system identification and formulation was first carried out for propofolnd etomidate infusion in rats. Data collected by Vijn and Sneyd of calculated BSR time courses after 10-second bolus doses (8 mg kg$^{-1}$ propofol, 3.5 mg kg$^{-1}$ etomidate) were used for the estimation of pharmacokinetics models. These estimated parameters were used to design the closed-loop controller (see below) in order to yield a critically damped system response, that is, the fastest rise times without overshoot. Table 1 shows the system parameters for both propofol and etomidate infusion in rats.

Table 1. The rate constants (given in min$^{-1}$) and scaling factor for the pharmacokinetics model estimation and the optimized deterministic controller gains for the drug-specific rat models.

|  | **Propofol** | **Etomidate** |
|---|---|---|
| $K_{12}$ | 0.01557 | 0.01547 |
| $K_{21}$ | 0.9599 | 0.6703 |
| $K_{10}$ | 4.552 | 1.993 |
| $\sigma$ | 0.5885 | 0.8394 |
| $K_p$ | 9.423 | 1.275 |
| $K_i$ | 0.1508 | 0.01425 |

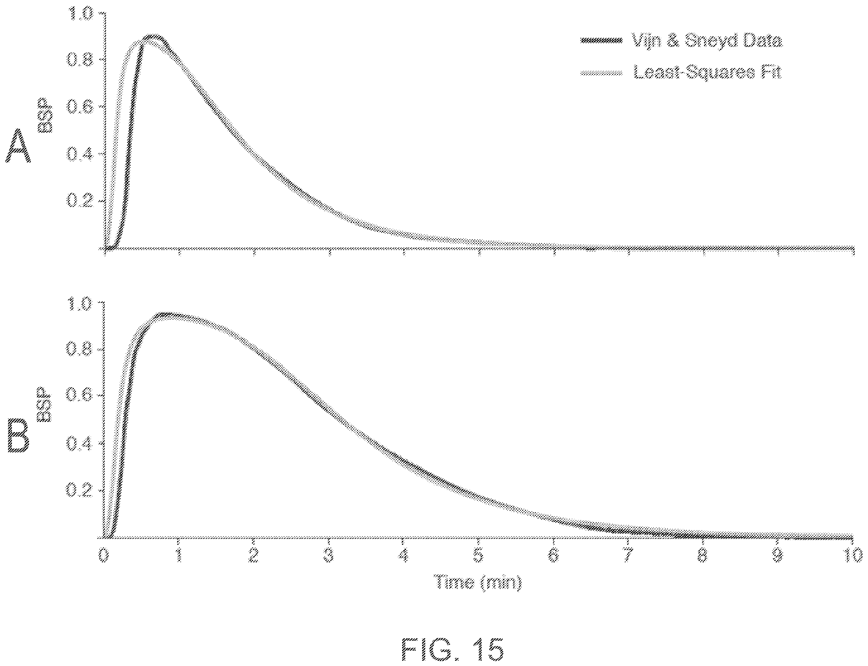

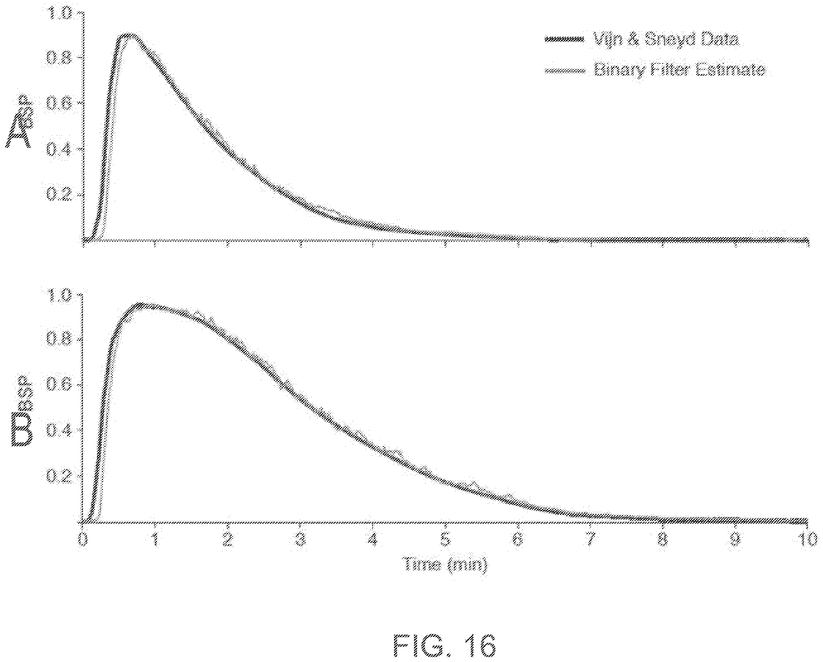

Figs. 15A and 15B show the simulated BSP time courses for these estimated pharmacokinetic systems compared to the Vijn and Sneyd reported data. To truly test the feasibility of the closed-loop system in accordance with the present invention, the binary filter would need to be added to the closed-loop system to emulate the stochastic nature of its practical application. The algorithm was implemented and tested on the Vijn and Sneyd data to ensure it was accurately outputting a dynamic estimate of $p_t$. At each 1-second interval of the Vijn and Sneyd data, a Bernoulli process of 200 binary integers was generated, simulating an EEG signal partitioned into intervals and thresholded. This vector was used as input to the binary filter algorithm, which output one updated $\hat{p}_{t|t}$ per second. Based on the ability of the algorithm to accurately and dynamically estimate the Vijn and Sneyd BSR curve, as is evident in Figs. 16A-B, it was incorporated into the control loop to model a stochastic control system.

The optimized controller was tested at six target BSP values, equally spaced on the interval [0.15, 0.9]. First, the system response was simulated with a deterministic feedback signal; the numerically evaluated $x_{2t}$ was simply transformed to $pt$ and fed back at each time interval, without the use of the binary filter. For these six targets, the simulated closed-loop system performed with an average rise time $(t_{90\%} - t_{10\%})$ of 1.319 minutes for propofol infusion and 3.031 minutes for etomidate infusion and, as predicted by the controller design theory, no overshoot in either model. To provide a more realistic simulation in this deterministic feedback scenario, infusion and 9.411 minutes for etomidate infusion, with no target overshoot. Fig. 19A-D' shows the system response of the human models in these three feedback scenarios.

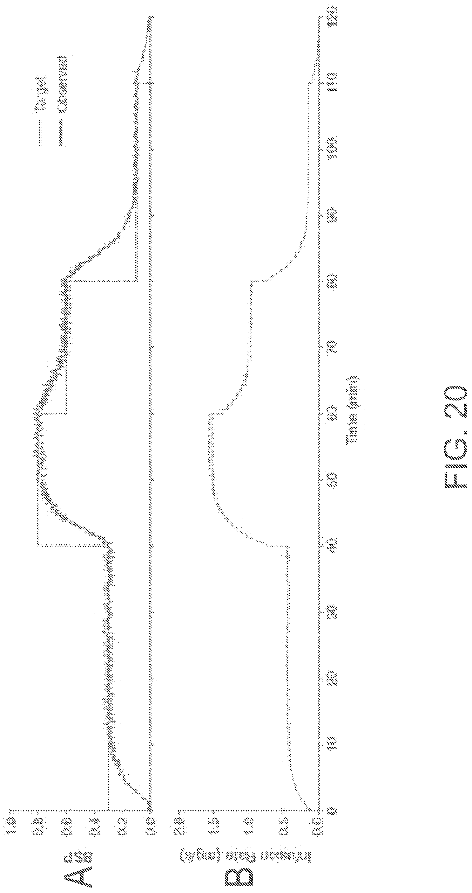

Finally, to simulate the BSP-targeting scheme that could be applied in the clinical setting, the closed-loop system was tested with a changing target, driving the human model deeper into burst suppression, and ending with the patient coming out of burst suppression (see Fig. 20).

With respect to the pharmacokinetics model estimation, data for the rat models was taken from the CLAD system study of Vijn and Sneyd, which presented smoothed time courses of calculated BSR in rats after a 10-second bolus dose (8 mg kg$^{-1}$ propofol, 3.5 mg kg$^{-1}$ etomidate). For the human models, existing population pharmacokinetic models were calibrated for a 30-year-old, 180-cm, 70-kg male. For propofol, the population model of Schnider TW, Minto CF, Gambus PL, Andresen C, Goodale DB, et al. (1998) The influence of method of administration and covariates on the pharmacokinetics of propofol in adult volunteers. Anesthesiology 88: 1170-82, was used to numerically evaluate the effect-site concentration during and after a 10-second bolus of 250 mg kg$^{-1}$. Since a patient may not enter a state of burst suppression the instant the effect-site concentration is nonzero, the concentration trace was shifted negatively and transformed to *pt* such that the patient peaked at a BSP of 0.7, and came out of burst suppression 7 minutes after the initiation of the dose. For etomidate, the population model of Arden JR, Holley FO, Stanski DR (1986). Increased sensitivity to etomidate in the elderly: initial distribution versus altered brain response. Anesthesiology 65: 19-27, was used to numerically evaluate the effect-site concentration during and after a 10-second bolus of 110 mg kg$^{-1}$. The concentration trace was shifted negatively and transformed to *pt* such that the patient peaked at a BSP of 0.7, and came out of burst suppression 12 minutes after the initiation of the dose.

To fit a two-compartment pharmacokinetic model to this data, a Matlab function was created to calculate the mean squared error between these BSP time courses and a BSP time course numerically evaluated from a given set of two-compartment-model parameters $k_{10}$, $k_{12}$, $k_{21}$, and $\sigma$. Matlab's Optimization Toolbox was used to optimize these four parameters of the pharmacokinetic model to minimize infusion and 9.411 minutes for etomidate infusion, with no target overshoot. Fig. 19A-D' shows the system response of the human models in these three feedback scenarios.

Finally, to simulate the BSP-targeting scheme that could be applied in the clinical setting, the closed-loop system was tested with a changing target, driving the human model deeper into burst suppression, and ending with the patient coming out of burst suppression (see Fig. 20).

With respect to the pharmacokinetics model estimation, data for the rat models was taken from the CLAD system study of Vijn and Sneyd, which presented smoothed time courses of calculated BSR in rats after a 10-second bolus dose (8 mg kg$^{-1}$ propofol, 3.5 mg kg$^{-1}$ etomidate). For the human models, existing population pharmacokinetic models were calibrated for a 30-year-old, 180-cm, 70-kg male. For propofol, the population model of Schnider TW, Minto CF, Gambus PL, Andresen C, Goodale DB, et al. (1998) The inuence of method of administration and covariates on the pharmacokinetics of propofol in adult volunteers. Anesthesiology 88: 1170-82, was used to numerically evaluate the effect-site concentration during and after a 10-second bolus of 250 mg kg$^{-1}$. Since a patient may not enter a state of burst suppression the instant the effect-site concentration is nonzero, the concentration trace was shifted negatively and transformed to *pt* such that the patient peaked at a BSP of 0.7, and came out of burst suppression 7 minutes after the initiation of the dose. For etomidate, the population model of Arden JR, Holley FO, Stanski DR (1986). Increased sensitivity to etomidate in the elderly: initial distribution versus altered brain response. Anesthesiology 65: 19-27, was used to numerically evaluate the effect-site concentration during and after a 10-second bolus of 110 mg kg$^{-1}$. The concentration trace was shifted negatively and transformed to *pt* such that the patient peaked at a BSP of 0.7, and came out of burst suppression 12 minutes after the initiation of the dose.

To fit a two-compartment pharmacokinetic model to this data, a Matlab function was created to calculate the mean squared error between these BSP time courses and a BSP time course numerically evaluated from a given set of two-compartment-model parameters $k_{10}$, $k_{12}$, $k_{21}$, and $\sigma$. Matlab's Optimization Toolbox was used to optimize these four parameters of the pharmacokinetic model to minimize this mean squared error, and yield an estimate of the best-fit rate constants and scaling factor.

In summary, the present invention provides an electroencephalogram (EEG) marker, which, in examples not being according to the claimed invention, may be related directly to the effect of an anesthetic drug, that can be computed in real-time and used to generate an error signal. This error signal or the difference between the measured marker value and the marker value targeted can be used to maintain the desired anesthetic state. In one example not being according to the claimed invention, the error signal is processed in real-time to adjust the rate of drug administration from a computer-controlled infusion device. If the pharmacokinetics of the underlying system and the response of the closed-loop controller are well characterized, it is possible to control the EEG marker and maintain the brain in the specified anesthetic state for as long as desired.

In accordance with the present invention, a new metric, for example, burst suppression probability (BSP), is utilized in new and different methodologies not contemplated in traditional systems relying on old metrics, such as burst suppression ratio (BSR). In addition, a system and method is provided for converting EEG bursts and suppressions into binary time series in real-time. The binary time series serves as an input to a burst suppression probability (BSP) algorithm. The BSP algorithm provides a second-to-second estimate of the brain's state of burst suppression using a state space model for binary and point process observations. An analysis paradigm is provided to estimate model parameters using an expectation maximization (EM) algorithm. The EM algorithm provides confidence intervals to facilitate formal statistical comparisons between the BSP estimated at any two time points.

Additionally, the present invention provides a closed-loop system for monitoring and controlling administration of anesthetic compounds and/or a current or desired state of a patient receiving the anesthetic compounds. The closed-loop system of the present invention can be designed to utilize burst suppression and the BSP as control mechanisms.

Multi-compartment, burst-suppression-specific models for various drugs have been implanted using pharmacokinetic population models. More particularly, drug-specific proportional-integral (PI) controllers have been designed to maintain a specific, BSP target level. Within this control system, an error signal can be reliably computed using a binary filter algorithm of the present invention to estimate the BSP from EEG recordings. Additionally, the present invention provides a system and method for determining the state of a patient's brain under anesthesia using readily-available monitoring information, such as from a patient's electroencephalography (EEG).

The present invention also provides a two-compartment model composed of a central plasma compartment and an auxiliary effect-site compartment connected by a first-order transfer process and may ignore any other peripheral drug distribution compartments for simplicity and effectiveness, as desired. The model can be used in a closed-loop system to advantageously utilize a binary filter algorithm to convert EEG signals in real-time into an estimate of the BSP. Again, the greater the BSP the greater the level of suppression, and hence the BSP provides a specific quantitative measure of the depth of the induced medical coma. A mathematical model that parametrically relates anesthetic infusion dynamics to the burst suppression probability is provided. Signal processing algorithms for extracting burst suppression morphology from the EEG are also provided. The controller can be designed to regulate the BSP in real-time using feedback control based on the binary filter output.

The present disclosure recognizes that anesthetic compounds induce different signatures in physiological characteristics of the patient under anesthesia and aids interpretation of such information. For example, the present disclosure is adaptable for use with a diverse list of clinical applications including, for example, control of medical coma, control of intensive care unit (ICU) sedation, control of general anesthesia in the operating room (OR), control of sedation for outpatient procedures, and the like. Unlike previous attempts to control depth of anesthesia and other CLAD systems, this disclosure is envisioned for use in conjunction with the medical judgment of a clinician, by providing an "autopilot" to help maintain specific physiologically-defined brain states selected by the clinician. This CLAD system thereby enhances the ability of the clinician to manage the care of the patient. Using the physiological characteristics and signatures associated with the selected anesthetic compound, the present disclosure aids the correlation of the physiological characteristics and signatures to a state of the patient's brain. Further still, the present invention provides systems and method for actively controlling and/or inducing an active arousal in a patient under the influence of anesthetic compounds.

In accordance with another aspect of the present invention, an automated system is provided for the maintenance of burst suppression to achieve a precise or desired patient state. The present invention can be used to identify and track burst suppression with respect to particular in disease states, such as hypothermia, general anesthesia, medical coma, and the like. As such, the present invention can be used, for example, to implement a rational approach to the use of hypothermia for neuroprotective purposes during cardiopulmonary bypass, total circulatory arrest and treatment of post-anoxic encephalopathy.

More specifically, the above-described closed-loop system can advantageously utilize the binary filter algorithm described above to convert the EEG in real-time into an estimate of the BSP. Again, the greater the BSP the greater the level of suppression, and hence the BSP provides a specific quantitative measure of the depth of the induced medical coma. A mathematical model that parametrically relates anesthetic infusion dynamics to the burst suppression probability is provided. Signal processing algorithms for extracting burst suppression morphology from the EEG are also provided. The controller can be designed to regulate the BSP in real-time using feedback control based on the binary filter output.

In addition to the above-described capabilities, the closed-loop system can utilize an extended algorithm for a complete multi-drug solution and active awaking. Specifically, the present invention recognizes that anesthetic compounds induce different signatures in physiological characteristics of the patient under anesthesia and aids interpretation of physiological characteristics and signatures therein based on a selected anesthesia compound. Using the physiological characteristics and signatures associated with the selected anesthesia compound, the present invention aids in relating of the physiological characteristics and signatures to a state of the patient's brain, such as described in detail in co-pending application PCT/US12/36854, entitled, "SYSTEM AND METHOD FOR TRACKING BRAIN STATES DURING ADMINISTRATION OF ANESTHESIA" published as WO 2012/154701 A1.

The various configurations presented above are merely examples and are in no way meant to limit the scope of this disclosure. Variations of the configurations described herein will be apparent to persons of ordinary skill in the art, the claimed invention being defined by the appended claims. In particular, features from one or more of the above-described configurations may be selected to create alternative configurations comprised of a sub-combination of features that may not be explicitly described above. In addition, features from one or more of the above-described configurations may be selected and combined to create alternative configurations comprised of a combination of features which may not be explicitly described above. Features suitable for such combinations and sub-combinations would be readily apparent to persons skilled in the art upon review of the present application as a whole.

**Claims**

1. A system (410) for monitoring a patient experiencing an administration of at least one drug having anesthetic properties, the system (410) comprising:

   a plurality of sensors configured to acquire at least EEG data from the patient;
   a user interface (422) configured to receive an indication of at least one of a characteristic of the patient and the at least one drug having anesthetic properties;
   at least one processor **characterized in that** the at least one processor is configured to:

   receive the EEG data from the plurality of sensors and the indication from the user interface;
   assemble the EEG data from the plurality of sensors into sets of EEG data forming time-series of EEG data;
   analyze the sets of EEG data using thresholding to generate binary time series from the sets of EEG data and using a burst suppression probability (BSP) algorithm implementing a state-space model to generate a time-series of burst suppression probabilities from the binary time series, wherein burst suppression probabilities are related to the patient brain's instantaneous likelihood of being in a suppressed state;
   determine, using the time-series of burst suppression probabilities, signature profiles consistent with the administration of at least one drug having anesthetic properties;
   identify, using the signature profiles, at least one of a current state and a predicted future state of the patient induced by the at least one drug, based on the indication; and
   generate a report indicating at least one of the current state and the predicted future state of the patient induced by the drug.

2. The system (410) of claim 1 wherein the processor is configured to use the current state, determined signature profiles, and the indication in a model to determine the predicted future state of the patient.

3. The system (410) of claim 1 wherein indication of at least one of a characteristic of the patient and the at least one drug having anesthetic properties includes at least one of an age of the patient, a drug selecting from the list consisting essentially of Propofol, Etomidate, Barbiturates, Thiopental, Pentobarbital, Phenobarbital, Methohexital, Benzodiazepines, Midazolam, Diazepam, Lorazepam, Dexmedetomidine, Ketamine, Sevoflurane, Isoflurane, Desflurane, Remifenanil, Fentanyl, Sufentanil, Alfentanil, and drug administration information including at least one of drug timing, drug dose, and drug administration rate.

4. The system (410) of claim 1 wherein the processor is further configured to generate commands for a drug delivery system to direct the administration of the least one drug by the drug delivery system to the patient to attain the predicted future state.

5. The system (410) of claim 1 wherein the processor is configured to implement a dynamic processing method to characterize the patient as exhibiting a predetermined behavioral dynamic, and wherein the behavioral dynamic includes at least one of a loss consciousness and recovery of consciousness.

6. A method (600) for operating a system monitoring a patient experiencing an administration of at least one drug having anesthetic properties, the method comprising:

   arranging a plurality of sensors configured to acquire at least EEG data from a patient; and
   controlling the system monitoring the patient to:

   receive (604) the EEG data from the plurality of sensors and an indication of at least one of a characteristic of the patient and the at least one drug having anesthetic properties from a user interface;
   assemble the EEG data from the plurality of sensors into sets of EEG data forming time-series of EEG data;
   analyze the sets of EEG data using thresholding to generate binary time series from the sets of EEG data and using a burst suppression probability (BSP) algorithm implementing a state-space model to generate a time-series of burst suppression probabilities from the binary time series, wherein burst suppression probabilities are related to the patient brain's instantaneous likelihood of being in a suppressed state;
   determine, using the time-series of burst suppression probabilities, signature profiles consistent with the administration of at least one drug having anesthetic properties;
   identify, using the signature profiles, at least one of a current state and a predicted future state of the patient, based on the indication; and

generate (614) a report including information regarding at least one of the current state and the predicted future state of the patient induced by the drug.

7. The method (600) of claim 6 wherein the system monitoring the patient is further controlled to use the current state, determined signature profiles, and the indication in a model to determine the predicted future state of the patient.

**Patentansprüche**

1. System (410) zum Überwachen eines Patienten, der eine Verabreichung von mindestens einem Medikament mit anästhetischen Eigenschaften erfährt, umfassend:

eine Vielzahl von Sensoren, die so konfiguriert sind, dass sie zumindest EEG-Daten des Patienten erfassen; eine Benutzerschnittstelle (422), die so konfiguriert ist, dass sie eine Angabe von mindestens einem Merkmal des Patienten und dem mindestens einen Medikament mit anästhetischen Eigenschaften empfängt; mindestens einen Prozessor, **dadurch gekennzeichnet, dass** der mindestens eine Prozessor konfiguriert ist, um:

die EEG-Daten von der Vielzahl von Sensoren und die Angabe von der Benutzerschnittstelle zu empfangen; die EEG-Daten von der Vielzahl von Sensoren zu EEG-Datensätzen zusammenstellen, die Zeitreihen von EEG-Daten bilden; die EEG-Datensätze zu analysieren unter Verwendung von Schwellenwerten, um binäre Zeitreihen aus den EEG-Datensätzen zu erzeugen, und unter Verwendung eines Algorithmus zur Burstunterdrückungswahrscheinlichkeit (BSP), der ein Zustandsraummodell implementiert, um eine Zeitreihe von Burstunterdrückungswahrscheinlichkeiten aus den binären Zeitreihen zu erzeugen, wobei Burstunterdrückungswahrscheinlichkeiten mit der momentanen Wahrscheinlichkeit des Patientengehirns, sich in einem unterdrückten Zustand zu befinden, in Beziehung stehen ; unter Verwendung der Zeitreihe von Burstunterdrückungswahrscheinlichkeiten Signaturprofile zu bestimmen, die mit der Verabreichung mindestens eines Medikaments mit anästhetischen Eigenschaften übereinstimmen, unter Verwendung der Signaturprofile mindestens einen aktuellen Zustand und einen vorhergesagten zukünftigen Zustand des Patienten, der durch das mindestens eine Medikament hervorgerufen wird, auf der Grundlage der Angabe zu identifizieren; und einen Bericht zu erstellen, der den aktuellen Zustand und/oder den voraussichtlichen zukünftigen Zustand des Patienten angibt, der durch das Medikament hervorgerufen wurde.

2. System (410) nach Anspruch 1, wobei der Prozessor so konfiguriert ist, dass er den aktuellen Zustand, die ermittelten Signaturprofile und die Angabe in einem Modell verwendet, um den vorhergesagten zukünftigen Zustand des Patienten zu bestimmen.

3. System (410) nach Anspruch 1, wobei die Angabe mindestens eines Merkmals des Patienten und des mindestens einen Medikaments mit anästhetischen Eigenschaften mindestens eines der folgenden Merkmale umfasst: Alter des Patienten, ein Medikament, das aus der Liste ausgewählt wird, die im Wesentlichen aus Propofol, Etomidat, Barbituraten, Thiopental, Pentobarbital, Phenobarbital, Methohexital, Benzodiazepine, Midazolam, Diazepam, Lorazepam, Dexmedetomidin, Ketamin, Sevofluran, Isofluran, Desfluran, Remifenanil, Fentanyl, Sufentanil, Alfentanil, und Informationen über die Verabreichung des Medikaments, einschließlich mindestens einer der folgenden Informationen: Zeitpunkt der Verabreichung des Medikaments, Medikamentendosis und Verabreichungsrate.

4. System (410) nach Anspruch 1, wobei der Prozessor ferner so konfiguriert ist, dass er Befehle für ein Arzneimittelverabreichungssystem erzeugt, um die Verabreichung des mindestens einen Arzneimittels durch das Arzneimittelverabreichungssystem an den Patienten zu steuern, um den vorhergesagten zukünftigen Zustand zu erreichen.

5. System (410) nach Anspruch 1, wobei der Prozessor so konfiguriert ist, dass er ein dynamisches Verarbeitungsverfahren implementiert, um den Patienten so zu charakterisieren, dass er eine vorbestimmte Verhaltensdynamik aufweist, und wobei die Verhaltensdynamik mindestens eines von Bewusstseinsverlust und Wiedererlangung des Bewusstseins umfasst.

6. Verfahren (600) zum Betreiben eines Systems zur Überwachung eines Patienten, der eine Verabreichung von mindestens einem Medikament mit anästhetischen Eigenschaften erfährt, umfassend:

Anordnen einer Vielzahl von Sensoren, die so konfiguriert sind, dass sie zumindest EEG-Daten von einem Patienten erfassen; und

Steuern des Systems zur Überwachung des Patienten, um:

die EEG-Daten von der Vielzahl von Sensoren und eine Angabe von mindestens einer Eigenschaft des Patienten und des mindestens einen Medikaments mit anästhetischen Eigenschaften von einer Benutzerschnittstelle zu empfangen (604);

die EEG-Daten von der Vielzahl der Sensoren zu EEG-Datensätzen zusammenstellen, die Zeitreihen von EEG-Daten bilden;

die EEG-Datensätze unter Verwendung von Schwellenwerten zu analysieren, um binäre Zeitreihen aus den EEG-Datensätzen zu erzeugen, und einen Algorithmus zur Burstunterdrückungswahrscheinlichkeit (BSP) zu verwenden, der ein Zustandsraummodell implementiert, um eine Zeitreihe von Burstunterdrückungswahrscheinlichkeiten aus den binären Zeitreihen zu erzeugen, wobei die Burstunterdrückungswahrscheinlichkeiten mit der momentanen Wahrscheinlichkeit des Patientengehirns, sich in einem unterdrückten Zustand zu befinden, in Beziehung stehen;

Signaturprofile zu bestimmen, die mit der Verabreichung mindestens eines Medikaments mit anästhetischen Eigenschaften übereinstimmen, unter Verwendung der Zeitreihe von Burstunterdrückungswahrscheinlichkeiten;

unter Verwendung der Signaturprofile mindestens einen aktuellen Zustand und einen vorhergesagten zukünftigen Zustand des Patienten auf der Grundlage der Angabe zu identifizieren; und

einen Bericht zu erstellen (614), der Informationen über den aktuellen Zustand und/oder den vorhergesagten zukünftigen Zustand des Patienten enthält, der durch das Medikament hervorgerufen wurde.

7. Verfahren (600) nach Anspruch 6, wobei das den Patienten überwachende System ferner so gesteuert wird, dass es den aktuellen Zustand, die ermittelten Signaturprofile und die Angabe in einem Modell verwendet, um den vorhergesagten zukünftigen Zustand des Patienten zu bestimmen.

**Revendications**

1. Système (410) pour surveiller un patient soumis à une administration d'au moins un médicament présentant des propriétés anesthésiques, le système (410) comprenant :

une pluralité de capteurs configurés pour acquérir au moins des données d'EEG du patient ;
une interface utilisateur (422) configurée pour recevoir une indication d'au moins une d'une caractéristique du patient et de l'au moins un médicament présentant des propriétés anesthésiques ;
au moins un processeur **caractérisé en ce que** l'au moins un processeur est configuré pour :

recevoir les données d'EEG provenant de la pluralité de capteurs et l'indication provenant de l'interface utilisateur ;
assembler les données d'EEG provenant de la pluralité de capteurs en ensembles de données d'EEG formant des séries chronologiques de données d'EEG ;
analyser les ensembles de données d'EEG au moyen d'un seuillage pour générer des séries chronologiques binaires à partir des ensembles de données d'EEG et au moyen d'un algorithme de probabilité de suppression de rafale (BSP) implémentant un modèle espace d'état pour générer une série chronologique de probabilités de suppression de rafale à partir de la série chronologique binaire, dans lequel les probabilités de suppression de rafale sont associées à la vraisemblance instantanée du cerveau du patient d'être dans un état supprimé ;
déterminer, au moyen de la série chronologique de probabilités de suppression de rafale, des profils de signature compatibles avec l'administration d'au moins un médicament présentant des propriétés anesthésiques ;
identifier, au moyen des profils de signature, au moins l'un d'un état actuel et d'un état futur prédit du patient induit par l'au moins un médicament, sur la base de l'indication ; et
générer un rapport indiquant au moins l'un de l'état actuel et de l'état futur prédit du patient induit par le médicament.

**2.** Système (410) selon la revendication 1 dans lequel le processeur est configuré pour utiliser l'état actuel, les profils de signature déterminés, et l'indication dans un modèle pour déterminer l'état futur prédit du patient.

**3.** Système (410) selon la revendication 1 dans lequel l'indication d'au moins l'une d'une caractéristique du patient et de l'au moins un médicament présentant des propriétés anesthésiques comporte au moins l'un d'un âge du patient, d'un médicament sélectionné dans la liste consistant essentiellement en propofol, étomidate, barbituriques, thiopental, pentobarbital, phénobarbital, méthohexital, benzodiazépines, midazolam, diazépam, lorazépam, dexmédétomidine, kétamine, sevoflurane, isoflurane, desflurane, rémifentanil, fentanyl, sufentanil, alfentanil, et des informations d'administration de médicament comportant au moins l'un de l'horaire de prise du médicament, de la dose du médicament, et de la vitesse d'administration du médicament.

**4.** Système (410) selon la revendication 1 dans lequel le processeur est en outre configuré pour générer des instructions pour un système de distribution de médicament pour diriger l'administration de l'au moins un médicament par le système de distribution de médicament au patient pour atteindre l'état futur souhaité.

**5.** Système (410) selon la revendication 1 dans lequel le processeur est configuré pour implémenter un procédé de traitement dynamique pour caractériser le patient comme laissant apparaître une dynamique comportementale prédéterminée, et dans lequel la dynamique comportementale comporte au moins l'une d'une perte de conscience et récupération de conscience.

**6.** Procédé (600) pour faire fonctionner un système surveillant un patient soumis à une administration d'au moins un médicament présentant des propriétés anesthésiques, le procédé comprenant :

l'agencement d'une pluralité de capteurs configurés pour acquérir au moins des données d'EEG d'un patient ; et
la commande du système surveillant le patient pour :

recevoir (604) les données d'EEG provenant de la pluralité de capteurs et une indication d'au moins l'une d'une caractéristique du patient et de l'au moins un médicament présentant des propriétés anesthésiques d'une interface utilisateur ;
assembler les données d'EEG provenant de la pluralité de capteurs en ensembles de données d'EEG formant des séries chronologiques de données d'EEG ;
analyser les ensembles de données d'EEG au moyen d'un seuillage pour générer des séries chronologiques binaires à partir des ensembles de données d'EEG et au moyen d'un algorithme de probabilité de suppression de rafale (BSP) implémentant un modèle espace d'état pour générer une série chronologique de probabilités de suppression de rafale à partir de la série chronologique binaire, dans lequel les probabilités de suppression de rafale sont associées à la vraisemblance instantanée du cerveau du patient d'être dans un état supprimé ;
déterminer, au moyen de la série chronologique de probabilités de suppression de rafale, des profils de signature compatibles avec l'administration d'au moins un médicament présentant des propriétés anesthésiques ;
identifier, au moyen des profils de signature, au moins l'un d'un état actuel et d'un état futur prédit du patient, sur la base de l'indication ; et
générer (614) un rapport comportant des informations concernant au moins l'un de l'état actuel et de l'état futur prédit du patient induit par le médicament.

**7.** Procédé (600) selon la revendication 6 dans lequel le système surveillant le patient est en outre commandé pour utiliser l'état actuel, les profils de signature déterminés, et l'indication dans un modèle pour déterminer l'état futur prédit du patient.

FIG. 1

Drug Infusion

Patient

Burst suppression level

Clinician

FIG. 2

FIG. 3

EP 2 906 112 B1

FIG. 4

34

500

START

502 PERFORM PRE-
PROCESSING ALGORITHM

504 PERFORM BRAIN STATE
ESTIMATION ALGORITHM

506 CORRELATE TO
CONFIDENCE INTERVAL

508 TRACK INDICATORS
RELATIVE TO DISEASE

510 OPERATE
CLOSED-LOOP
SYSTEM

END

FIG. 5

600

START

602 — SELECT DRUG AND/OR PATIENT PROFILE

604 — ACQUIRE PHYSIOLOGICAL DATA

606 — LOCALIZE ACQUIRED DATA (LAPLACIAN REFERENCING)

608 — PERFORM GLOBAL COHERENCE ANALYSIS

610 — CREATE SPECTROGRAM

612 — PERFORM PHASE-AMPLITUDE ANALYSIS

614 — PERFORM REAL-TIME ANALYSIS AND GENERATE REPORT

END

FIG. 6

FIG. 7

FIG. 8

FIG. 9A

FIG. 9B

$p_0$ $\longrightarrow$ $+$ $\bigcirc$ $\xrightarrow{e_t}$ | Controller and Pump | $\xrightarrow{u_t}$ | Compartmental Model | $\xrightarrow{p_t}$ | Burst Suppression EEG | $\xrightarrow{n_t}$ | Binary Filter | $\xrightarrow{p_t}$

$\hat{p}_{t|t}$
(or $\hat{x}_{t|t}$)

FIG. 10

FIG. 11

FIG. 12

FIG. 13

FIG. 14

FIG. 15

EP 2 906 112 B1

FIG. 16

FIG. 17

FIG. 18

FIG. 19

FIG. 20

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- US 2004079372 A1
- US 2004193068 A1
- US 1236854 W
- WO 2012154701 A1

## Non-patent literature cited in the description

- **BROWN EN ; LYDIC R ; SCHIFF ND.** General anesthesia, sleep, and coma. *New England Journal of Medicine,* 2010, vol. 363 (27), 2638-2650
- **BROWN EN ; PURDON PL ; VAN DORT CJ.** General anesthesia and altered states of arousal: a systems neuroscience analysis. *Annual Review of Neuroscience,* 2011, vol. 34, 601-628
- **PURDON PL ; PIERCE ET ; MUKAMEL EA ; PRERAU MJ ; WALSH JL ; WONG KFK ; SALAZAR-GOMEZ AF ; HARRELL PG ; SAMPSON A ; CIMENSER A.** Electroencephalogram signatures of loss and recovery of consciousness from propofol. *Proceedings of the National Academy of Sciences,* 19 March 2013, vol. 110 (12), E1142-51
- **CIMENSER A ; PURDON PL ; PIERCE ET ; WALSH JL ; SALAZAR-GOMEZ AF ; HARRELL PG ; TAVARES-STOECKEL C ; HABEEB K ; BROWN EN.** Tracking brain states under general anesthesia by using global coherence analysis. *Proceedings of the National Academy of Sciences of the United States of America,* 2011, vol. 108, 8832-8837
- **SOLT et al.** Methylphenidate Actively Induces Emergence from General Anesthesia. *Anesthesiology,* 2011, vol. 115, 791-803
- **CHEMALI et al.** Active Emergence from Propofol General Anesthesia Is Induced by Methylphenidate. *Anesthesiology,* 2012, vol. 116, 998-1005
- **CHEMALI JJ ; WONG KFK ; SOLT K ; BROWN EN.** A state-space model of the burst suppression ratio. *IEEE EMBC,* September 2011
- **SCHNIDER TW ; MINTO CF ; GAMBUS PL ; ANDRESEN C ; GOODALE DB et al.** The influence of method of administration and covariates on the pharmacokinetics of propofol in adult volunteers. *Anesthesiology,* 1998, vol. 88, 1170-82
- **ARDEN JR ; HOLLEY FO ; STANSKI DR.** Increased sensitivity to etomidate in the elderly: initial distribution versus altered brain response. *Anesthesiology,* 1986, vol. 65, 19-27
- **SCHNIDER TW ; MINTO CF ; GAMBUS PL ; ANDRESEN C ; GOODALE DB et al.** The inuence of method of administration and covariates on the pharmacokinetics of propofol in adult volunteers. *Anesthesiology,* 1998, vol. 88, 1170-82